# EUROPEAN PATENT SPECIFICATION

(11) **EP 1 066 315 B1**
(45) Date of publication and mention of the grant of the patent: **28.05.2008**
(21) Application number: 99913464.6
(22) Date of filing: 26.03.1999
(51) Int. Cl.: C07K 5/06, C07K 5/08, A61K 38/55, C07K 14/705, G01N 33/68, A01K 67/027

(54) **USE OF INHIBITORS OF MAMMALIAN ASPARAGINYL ENDOPEPTIDASE FOR MODULATING THE IMMUNE SYSTEM**
VERWENDUNG VON INHIBITOREN VON ASPARAGINYL-ENDOPEPTIDASE AUS SÄUGETIEREN ZUR MODULIERUNG DES IMMUNSYSTEMEN
UTILISATION D'INHIBITEURS D'ASPARAGINYL ENDOPEPTIDASE DE MAMMIFERE POUR LA MODULATION DU SYSTEME IMMUNITAIRE

(30) Priority: 26.03.1998 GB 9806442; 28.05.1998 US 86966 P
(43) Date of publication of application: 10.01.2001
(73) Proprietor: University of Dundee, Dundee DD1 4HN (GB)
(72) Inventor: WATTS, Colin, Newport-on-Tay DD6 8HX (GB)
(74) Representative: Miles, John Stephen
(86) International application number: PCT/GB1999/000963
(87) International publication number: WO 1999/048910

(56) References cited:
- CHEMICAL ABSTRACTS, vol. 128, no. 25, 22 June 1998 (1998-06-22) Columbus, Ohio, US; abstract no. 303708, W C BENJAMIN ET AL.: "Caspase activation in MCF7 cells responding to etoposide treatment" XP002113051 & MOL. PHARMACOL. , vol. 53, no. 3, 1998, pages 446-450,
- K K SHARMA ET AL.: "identification of new lens protease(s) using substrates having in vivo cleavage sites" BIOCHEMICAL AND BIOPHYSICAL RESEARCH COMMUNICATIONS., vol. 218, 1996, pages 365-370, XP002113049 ACADEMIC PRESS INC. ORLANDO, FL., US ISSN: 0006-291X
- J M CHEN ET AL.: "Cloning, isolation and characterization of mammalian legumain, an asparaginyl endopeptidase" JOURNAL OF BIOLOGICAL CHEMISTRY., vol. 272, no. 12, 21 March 1997 (1997-03-21), pages 80090-8098, XP002065429 AMERICAN SOCIETY OF BIOLOGICAL CHEMISTS, BALTIMORE, MD., US ISSN: 0021-9258 cited in the application
- B MANOURY T AL.: "An asparaginyl endopeptidase processes a microbial antigen for class II MHC presentation" NATURE., vol. 396, 17 December 1998 (1998-12-17), pages 695-699, XP002113050 MACMILLAN JOURNALS LTD. LONDON., GB ISSN: 0028-0836
- CHEMICAL ABSTRACTS, vol. 119, no. 13, 27 September 1993 (1993-09-27) Columbus, Ohio, US; abstract no. 133992, A A KEMBHAVI ET AL.: "The two cysteie endopeptidases of legume seeds; purification and characterization by use of specific fluorometric assays" XP002113052 & ARCH. BIOCHEM. BIOPHYS., vol. 303, no. 2, 1993, pages 208-213,
- CHEMICAL ABSTRACTS, vol. 121, no. 17, 24 October 1994 (1994-10-24) Columbus, Ohio, US; abstract no. 202741, M J ANDROLEWICZ ET AL.: "Human transporters associated with antigen processing possess a promiscuous binding site" XP002113053 & IMMUNITY, vol. 1, no. 1, 1994, pages 7-14,
- JINQ-MAY CHEN ET AL: 'Cloning, Isolation, and Characterization of Mammalian Legumain, an Asparaginyl Endopeptidase' JOURNAL OF BIOLOGICAL CHEMISTRY vol. 272, no. 12, 1997, US AMERICAN SOCIETY OF BIOLOGICAL CHEMISTS, BALTIMORE, MD., pages 8090 - 8098
- EMBO JOURNAL vol. 3, no. 4, 1984, GB OXFORD UNIVERSITY PRESS, SURREY., pages 869 - 872
- O'SULLIVAN D.M. ET AL: 'Structure of the Human Ia-Associated Invariant ( )-Chain Gene: Identification of5' Sequences Shared with Major Histocompatibility Complex Class II Genes' PROCEEDINGS OF THE NATIONAL ACADEMY OF SCIENCES OF USA. vol. 83, no. 12, 1986, US NATIONAL ACADEMY OF SCIENCE, WASHINGTON, DC., pages 4484 - 4488

## Description

The present invention relates to enzyme inhibition, and in particular to the inhibition of an enzyme that we call asparaginyl endopeptidase which we have found is involved in processing antigens, particularly microbial antigens, by the immune system.

The immune response to protein antigens involves a large number of individual gene products, and new ones are still being discovered. In some cases the gene products were known *per se,* but not known to be involved in the immune response.

An immune response may be raised towards foreign antigens, for example, antigens associated with microorganisms, or the immune response may be caused by a response to a self antigen (autoimmunity). In either case, there exists the need for methods and means for modulating the immune system and how it responds to these foreign and self antigens.

Because proteins must be proteolytically processed (ie partially degraded) before the T cells of the immune system can respond, one set of proteins of importance in an immune response are proteolytic enzymes (proteases). It is typically proteolysed proteins (ie protein fragments) which are presented by antigen presenting cells on MHC Class I or Class II molecules.

Several different aspartic and cysteine proteases are thought to be involved in invariant chain and antigen processing (see, for example, Fineschi & Miller (1997) Trends Biochem. Sci. 22, 377-382; and Chapman (1998) Curr. Op. Immunol. 10, 93-102). For example, there is now good evidence that cathepsin S plays a role in the final stages of invariant chain processing (see, for example, Riese et al (1996) Immunity 4, 357-366; and Villadangos et al (1997) J. Exp. Med. 186, 549-560).

WO 97/40066 relates to the suppression of immune response by inhibiting cathepsin S.

We have now found, surprisingly, a further protease which is involved in processing protein antigens. The enzyme has asparaginyl endopeptidase (AEP) activity and, until the work described in this patent application, was not known to be involved in the immune response.

An enzyme with AEP activity was first detected in legumes and was called "legumain". Enzymes with AEP activity have also been detected in the blood fluke *Schistosoma mansoni* where it is involved in degrading haemoglobin (haemoglobinase) and, more recently, in mammals from which the cDNA has also been cloned (Chen et al (1997) J. Biol. Chem. 272, 8090-8096). For the avoidance of doubt, and in the event that different names are used, the animal enzymes with asparaginylendopeptidase activity may interchangeably be referred to as "asparaginyl endopeptidase", "AEP", "legumain" or "haemoglobinase". It will be appreciated that any particular animal, such as a human, may have more than one enzyme with asparaginyl endopeptidase activity, and that, in the context of the invention now described, an inhibitor of AEP may inhibit one or more forms of AEP.

Androlewicz et al (1994) immunity 1, 7-14 relates to human transporters associated with antigen processing.

A first aspect of the invention provides use of a selective inhibitor of asparaginyl endopeptidase in the manufacture of a medicament for use as an immunosuppresive agent. The medicament can be for the treatment of an autoimmune, allergic, hypersensitivity or transplant rejection disorder. The selective inhibitor is at least ten times more potent for AEP than for another cysteine protease that does not have asparaginyl endopeptidase activity.

We have found that an enzyme with AEP activity is present in the lysosomes of B cells and is involved in degrading certain proteins. It is fragments of a protein, produced following proteolytic degradation, which are loaded onto and presented by Class II MHC molecules. Inhibition of AEP activity has been shown to interfere with the degradation of certain asparagine-containing proteins and to substantially inhibit the loading and presentation of peptides on Class II MHC molecule-containing cells (and to substantially inhibit T cell activation).

Furthermore, we have shown that AEP can degrade the so-called "invariant chain" which is a dedicated chaperone for class II MHC which must be removed by proteolysis before any peptide can be loaded (Cresswell (1996) Cell 84, 505). There are two alternatively spliced forms of the invariant chain, usually known as p31 and p41. Inhibition of AEP is believed to reduce the competency of Class II MHC molecules for binding antigenic peptides and reduce the presentation of antigenic peptides by Class II MHC molecules. Thus, inhibitors of AEP are likely to be immunosuppressive agents.

An enzyme with AEP activity is readily able to cleave the substrate Z-Ala-Ala-Asn-7-(4-methyl)coumarylamide to release a fluorescent product, where Z is benzyloxy carbonyl.

By an "inhibitor of asparaginyl endopeptidase" we include any suitable inhibitor. The inhibitor may be an enzyme inhibitor such as a competitive inhibitor or it may be a non-competitive inhibitor.

AEP is a cysteine protease and so inhibitors which chemically react with the active cysteine residue are suitable and are, typically, irreversible inhibitors. Other amino acid residues, such as histidine, may be present at the active site and inhibitors which react with any active site residue are suitable.

In one embodiment of the invention the AEP inhibitor is a competitive inhibitor. Typically, the competitive inhibitor is a peptide comprising an asparagine-containing peptide. Suitably, the peptide is a peptide comprising a known AEP cleavage site but which, because of its affinity for AEP, can compete for another AEP cleavage site and substantially prevent cleavage at the said other cleavage site if present in sufficient concentration. Known cleavage sites for AEP in tetanus toxin are described in Chen et al (1997) J. Biol. Chem. 272, 8090-8098.

In a preferred embodiment of the invention the competitive inhibitor has the general structure (X₁)ₚ N(X₂)_{q} wherein X₁ and X₂ are amino acid residues, N is an asparagine residue, p is 3 to 6 and q is 1 to 3. Each of the p X₁ residues is independently selected and each of the q X₂ residues is independently selected.

Suitably, the competitive inhibitor is a peptide which comprises the peptide sequence Ala-Glu-Asn-Lys or, less preferably, Lys-Asn-Asn-Glu. Preferably, the competitive inhibitor is an N and C-terminal blocked peptide Ala-Glu-Asn-LysNH (AENK) or, less preferably, Lys-Asn-Asn-Glu-NH (KNNE).

As noted, and as is shown in Example 2, the class II MHC invariant chain is a substrate for AEP. Inspection of the invariant chain sequence indicates that any of the following asparagine residues may be processed by AEP: 71. 76, 132, 149 and 155 found in both p31 and p41 isoforms (p31 and p41 are two alternatively spliced forms of the invariant chain), and 219, 221, 240, 246 and 254 found only in the p41 isoform.

The sequence of the human invariant chain is given below. The p41 specific region is underlined. Asparagine residues that may be processed by AEP are highlighted in bold.

1
MHRRRS RSCREDQKPV MDDQRDLISN NEQLPMLGRR PGAPESKCSR
31 GALYTGFSIL VTLLLAGQAT TAYFLYQQQG RLDKLTVTSQ NLQLENLRMK
81 LPKPPKPVSK MRMATPLLMQ ALPMGALPQG PMQNATKYGN MTEDHVMHLL
131 QNADPLKVYP PLKGSFPENL THLKNTMETI DWKVFESWMH HWLLFEMSRH
181 SLEQKPTDAP PKVLTKCQEE VSHIPAVHPG SFRPKCDENG NYLPLQCYGS
231 IGYCWCVFPN GTEVPNTRSR GHHNCSESLE LEDPSSGLGV TKQDLGPVPM

The complete sequence of the mRNA (and encoded polypeptide) for the HLA-DR-associated invariant chain is described in Strubin et al (1984) EMBO J. 3, 869-872. The structure of the human Ia-associated invariant (gamma)-chain is described in O'Sullivan et. al (1986) Proc. Natl. Acad. Sci. USA 83, 4484-4488. Kudo et al. (1985) Nucl. Acids Res. 13, 8827-8841 describes the structure of the human gene encoding the invariant gamma chain of class II histocompatibility antigens.

Competitive inhibitors may be made based on the sequences which comprise, for example, two or three residues in each direction from the asparagine residue. Thus, for example, competitive inhibitors include residues 69 to 73, 68 to 74, 74 to 78, 73 to 79, 129 to 133, 128 to 134 and so on of the invariant chain. As is described in Example 2, both the p31 and p41 forms of the invariant chain are cleaved by AEP.

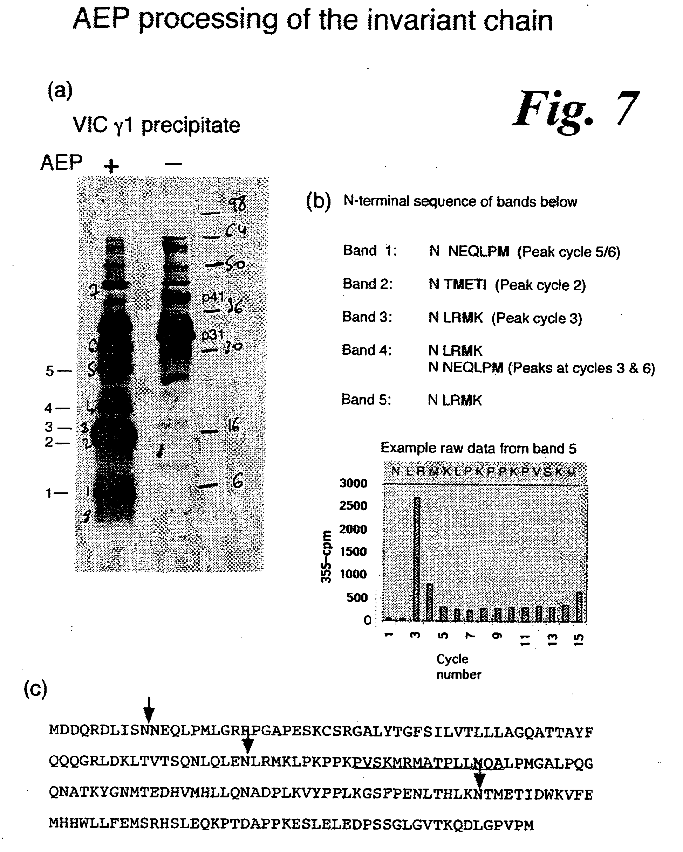

Figure 7 shows AEP processing of invariant chain.

Peptides may be synthesised by the Fmoc-polyamide mode of solid-phase peptide synthesis as disclosed by Lu et al (1981) J. Org. Chem. 46, 3433 and references therein. Temporary N-amino group protection is afforded by the 9-fluorenylmethyloxycarbonyl (Fmoc) group. Repetitive cleavage of this highly base-labile protecting group is effected using 20 % piperidine in N,N-dimethylformamide. Side-chain functionalities may be protected as their butyl ethers (in the case of serine threonine and tyrosine), butyl esters (in the case of glutamic acid and aspartic acid), butyloxycarbonyl derivative (in the case of lysine and histidine), trityl derivative (in the case of cysteine) and 4-methoxy-2,3,6-trimethylbenzenesulphonyl derivative (in the case of arginine). Where glutamine or asparagine are C-terminal residues, use is made of the 4,4'-dimethoxybenzhydryl group for protection of the side chain amido functionalities. The solid-phase support is based on a polydimethyl-acrylamide polymer constituted from the three monomers dimethylacrylamide (backbone-monomer), bisacryloylethylene diamine (cross linker) and acryloylsarcosine methyl ester (functionalising agent).

The peptide-to-resin cleavable linked agent used is the acid-labile 4-hydroxymethyl-phenoxyacetic acid derivative. All amino acid derivatives are added as their preformed symmetrical anhydride derivatives with the exception of asparagine and glutamine, which are added using a reversed N,N-dicyclohexyl-carbodiimide/1-hydroxybenzotriazole mediated coupling procedure. All coupling and deprotection reactions are monitored using ninhydrin, trinitrobenzene sulphonic acid or isotin test procedures. Upon completion of synthesis, peptides are cleaved from the resin support with concomitant removal of side-chain protecting groups by treatment with 95 % trifluoroacetic acid containing a 50 % scavenger mix. Scavengers commonly used are ethanedithiol, phenol, anisole and water, the exact choice depending on the constituent amino acids of the peptide being synthesised. Trifluoroacetic acid is removed by evaporation *in vacuo,* with subsequent trituration with diethyl ether affording the crude peptide. Any scavengers present are removed by a simple extraction procedure which on lyophilisation of the aqueous phase affords the crude peptide free of scavengers. Reagents for peptide synthesis are generally available from Calbiochem-Novabiochem (UK) Ltd, Nottingham NG7 2QJ, UK. Purification may be effected by any one, or a combination of, techniques such as size exclusion chromatography, ion-exchange chromatography and (principally) reverse-phase high performance liquid chromatography. Analysis of peptides may be carried out using thin layer chromatography, reverse-phase high performance liquid chromatography, amino-acid analysis after acid hydrolysis and by fast atom bombardment (FAB) mass spectrometric analysis.

In a further embodiment, the inhibitor may be a non-competitive or irreversible inhibitor. AEP activity can be blocked by high concentrations of inhibitors which block all cysteine proteases and some serine proteases. However, it is particularly preferred that the inhibitors are selective for AEP. Non-competitive (irreversible or covalent) inhibitors are more preferred than competitive inhibitors.

Generally, irreversible AEP inhibitors may be any of peptide aldehydes, peptide halomethyl ketones (particularly peptide chloromethyl ketones and peptide fluoromethyl ketones), peptide diazomethanes (diazomethyl ketones) or peptide vinyl sulphones, peptide (acyloxy)methanes, peptide N, O diacyl hydroxamates, peptide nitriles, peptide α-keto carbonyls, peptide ketomethylsulfonium salts, peptide epoxides or peptides which bind AEP and have a reactive group which will react with the active cysteine residue or other residue at the active site.

Suitably, the peptide sequence has the C-terminal residue as an asparagine residue to which is attached the group which reacts with the active site cysteine. The position of the asparagine residue in the peptide inhibitors (ie at the C terminus before the active group) is analogous to the P1 position at a cleavage site.

Thus, in general, an AEP inhibitor has the structure Bl-(X)ₙ-Asn-Q where Bl is a suitable N-terminal blocking group including, for example, acetyl or benzyloxycarbonyl; X is an amino acid residue; n is between 1 and 100, preferably between 1 and 50, more preferably between 1 and 10 and typically 2 to 6; Asn is an asparagine residue and Q is a group capable of reacting with the active site cysteine or other active site residue of AEP and, conveniently, forming a covalent complex thereby eliminating catalytic activity. It is preferred if the amino acid residues in the peptide Xₙ-Asn are in the L configuration and it is particularly preferred that, whether or not the Xₙ amino acid residues are in the L-configuration, that Asn is in the L-configuration. The amino acid residues X may be any naturally occurring amino acid residues or they may be residues which have non-natural side chains.

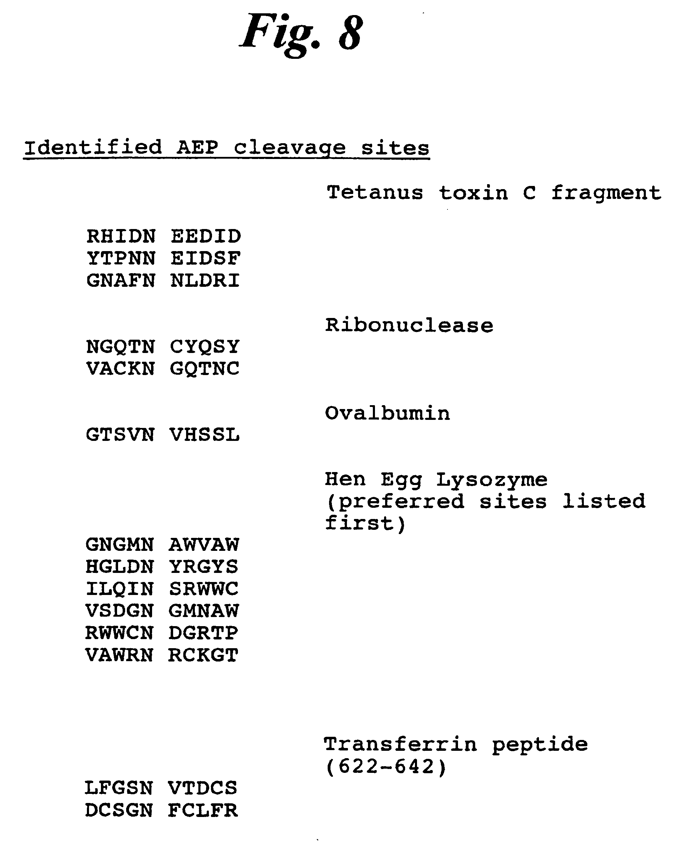

The peptide sequence before the C terminal asparagine and active group may be any suitable peptide sequence, but it will be appreciated that it is the sequence at a known AEP cleavage site. Cleavage sites for AEP are shown in, for example. Figures 7 and 8.

As noted, AEP cleaves both the p31 and p41 isoforms of the invariant chain. Non-competitive (or irreversible) inhibitors may readily be designed based on the residues N-terminal to the cleaved asparagine. As described above, any of the following asparagine residues may be processed by AEP: 71, 76, 132, 149 and 155 found in both p31 and p41, and 219, 221, 240, 246 and 254 found only in the p41 isoform. Thus, non-competitive inhibitors may be made which contain some of the amino acid residues N-terminal to these particular sites wherein the asparagine residue is linked to a group (Q) capable of reacting with the active site cysteine or other active site residue of AEP and, conveniently, forming a covalent complex thereby eliminating catalytic activity. Thus, AEP inhibitors may include Bl-Ser-Gln-Asn-Q; Bl-Leu-Glu-Asn-Q; Bl-Leu-Gln-Asn-Q: Bl-Pro-Glu-Asn-Q; Bl-Leu-Lys-Asn-Q (corresponding to asparagines 71, 76, 132, 149 and 155).

Two preferred inhibitors are Bl-Gln-Asn-Q and Bl-Glu-Asn-Q.

Other AEP inhibitors may include B1-Asp-Glu-Asn-Q; Bl-Asn-Gly-Asn-Q; Bl-Pbe-Pro-Asn-Q; Bl-Val-.Pro-Asn-Q; and Bl-His-His-Asn-Q. These inhibitors relate to Asn residues of the p41 isoform with numbers 219, 221, 240, 246 and 254, respectively.

Other cleavage sites in unrelated proteins and peptides are shown in Figure 8. These may be used to design suitable inhibitors as described above.

Typically, peptide aldehyde inhibitors have the structure:

Acetyl (or other blocking group)-Xₙ-Asparaginal

where X is an amino acid residue and n is between 1 and 100, preferably between 1 and 50, more preferably between 1 and 10 and typically 2 to 6.

A suitable peptide aldehyde includes acetyl-alanyl-glutamyl-asparagmal.

Elastatinal blocks AEP activity; however a more specific variant, in which the C terminal amino residue is replaced by asparagine, may be useful.

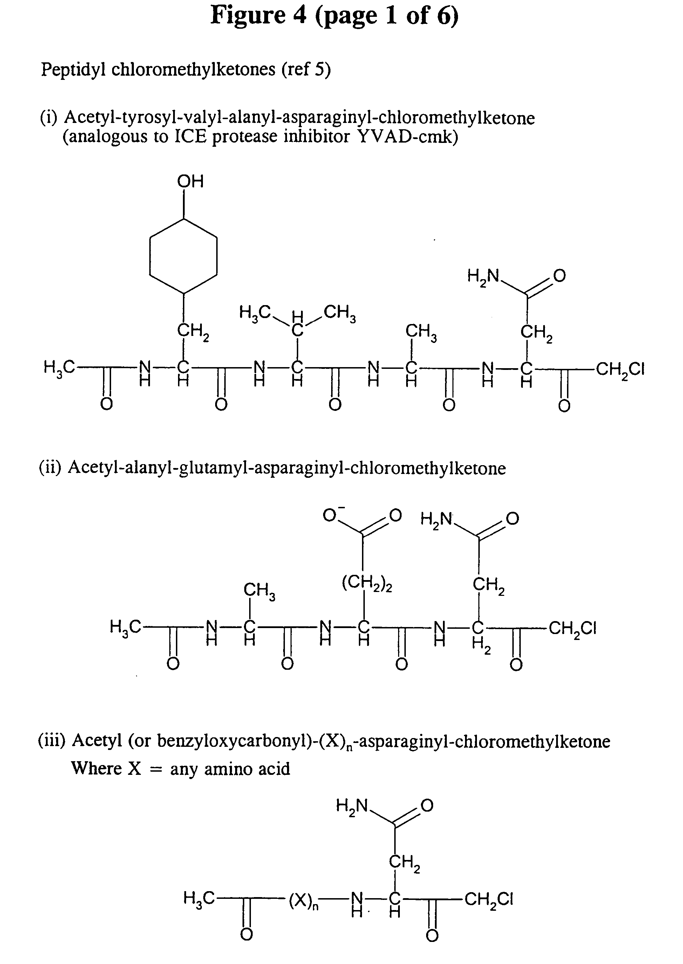

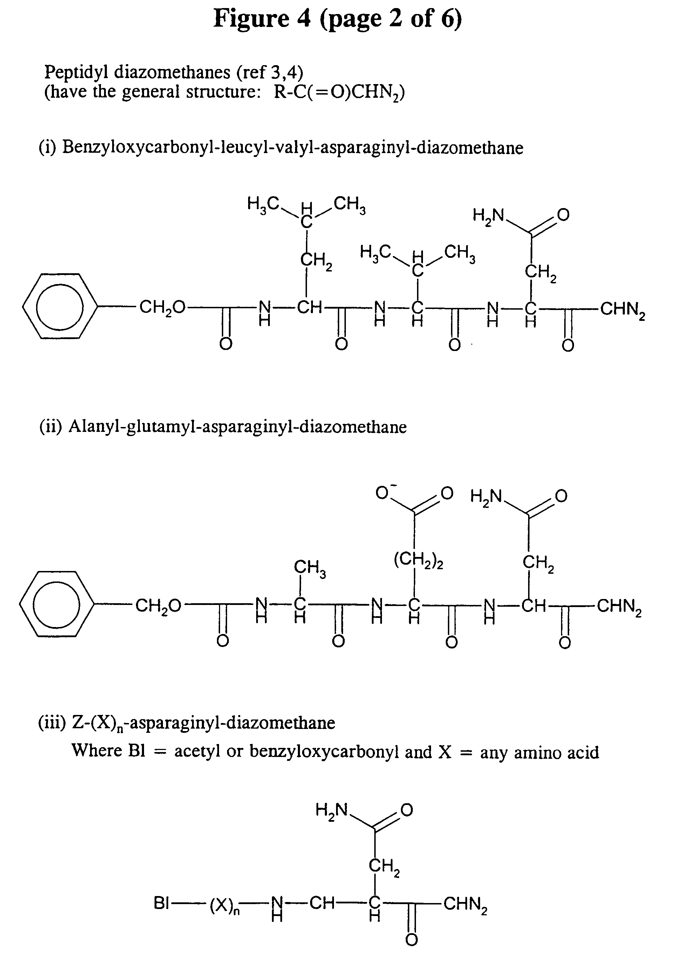

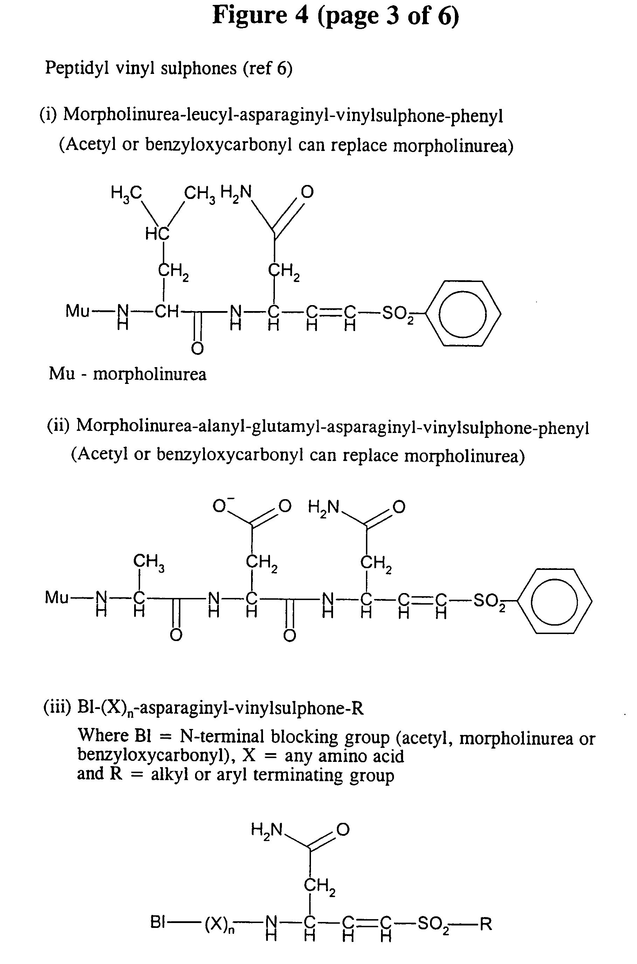

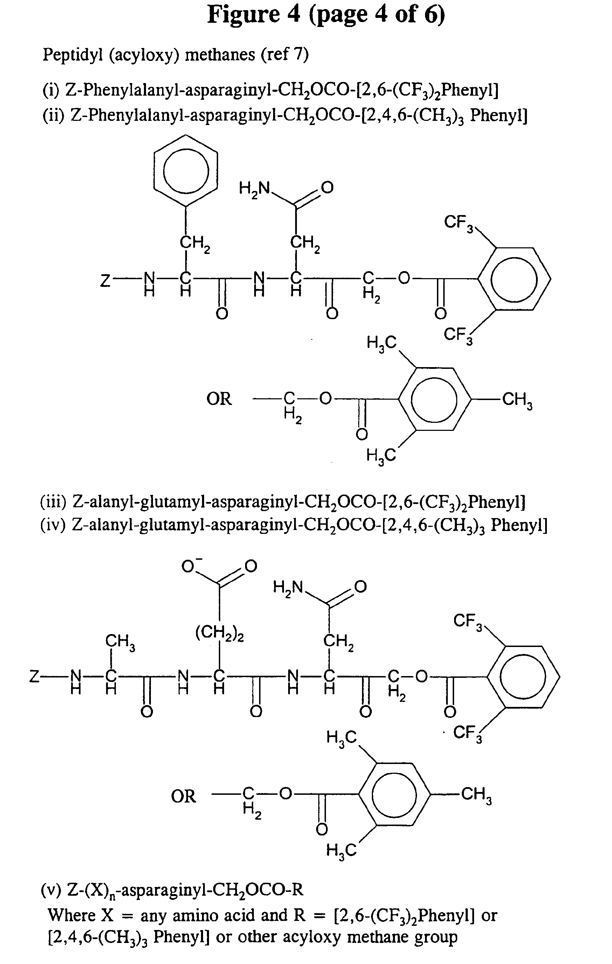

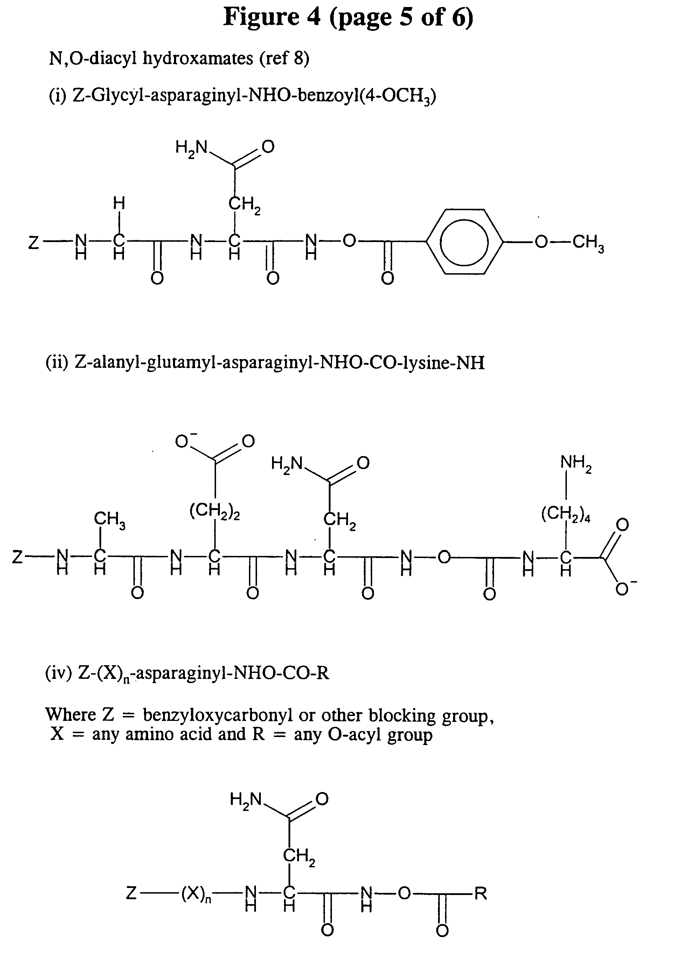

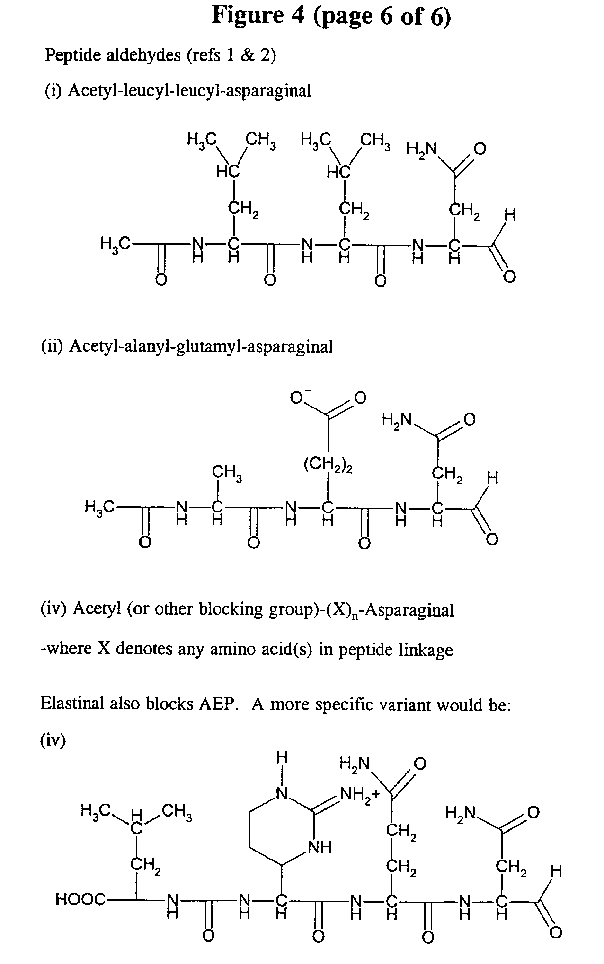

Figure 4 shows the structures of some useful inhibitors.

Typically, peptide chloromethylketone inhibitors have the structure.

Acetyl (or other blocking group)-Xₙ-asparaginyl-chloromethyl ketone where X and n are as above.

A suitable peptide includes acetyl-alanyl-glutamyl-asparaginyl-chloromethylketone and acetyl-tyrosyl-valyl-alanyl-asparaginyl-chloro methylketone (analogous to ICE protease inhibitor YVAD-cmk); see Figure 4 for further details.

Typically, peptide fluoromethylketone inhibitors have analogous structures to peptide chloromethylketone inhibitors except for the replacement of a chloro group with a fluoro group.

Typically, peptide diazomethane inhibitors have the structure

B1-(X)ₙ-asparaginyl-diazomethane

where B1 is any suitable blocking group including acetyl or benzyloxycarbonyl and X and n are as above. A diazomethane on the C-terminus of a peptide has a general structure R-C(O)CHN₂ where R represents the peptide.

A suitable peptide includes Bl-alanyl-asparaginyl-diazomethane or Bl-alanyl-glutamyl-asparaginyl-diazomethane.

Typically, peptide vinyl sulphone inhibitors have the structure

Bl-(X)ₙ-asparaginyl-vinyl sulphone-R

where Bl is an N-terminal blocking group such as acetyl or benzyloxycarbonyl, X and n are as above, and R is any suitable alkyl or aryl terminating group and includes C₁ to C₁₀ alkyl, phenyl, benzyl, naphthyl and the like.

A suitable peptide includes morpholinurea-leucyl-asparaginyl-vinyl sulphone-phenyl or morpholinurea-alanyl-glutamyl-asparaginyl-vinyl sulphone-phenyl.

Other suitable peptides include peptidyl(acyloxy) methanes and peptidyl N,O-diacylhydroxamates.

It will be appreciated from the foregoing that AEP inhibitors may be designed based on other cysteine protease inhibitors such as E-64 (1-trans-epoxysuccinyl-leucylamide (4-guanido)-butane), Leupeptin (acetyl-leucyl-leucyl-arginal), Antipain ([(S)-1-carboxy-2-phenyl]-carbamoyl-Arg-Val-arginal), Elastinal (N-[(S)-1-carboxy-isopentyl)-carbamoyl-α-(2-iminohexahydro-4(S)-pyrimidyl]-L-glycyl-L-glutaminyl-L-alaninal), TLCK (tosyllysylchloromethylketone) and TPCK (tosylphenylalanylchloromethylketone), for example by introduction of an asparaginyl residue at an appropriate place in place of an existing amino acid residue.

Vinyl sulphone inhibitors of cysteine proteases are described in Palmer et al (1995) J. Med. Chem. 38, 3193-3196. Peptide aldehyde inhibitors of cysteine proteases are described in Thomson Methods Enzymol. 46, 220 - and Vinitsky et al (1994) J. Biol. Chem. 269, 29860-29866 639-648. Peptide diazomethanes as inhibitors of cysteine proteases are described in Shaw (1994) Methods Enzymol. 244, 649-656 and Shaw & Green (1981) Methods Enzymol. 80, 820-826. Peptide (aryloxy) methanes as inhibitors of cysteine proteases are described in Krantz (1994) Methods Enzymol. 244, 656-671. Peptide N,O-diarylhydroxamates as inhibitors of cysteine proteases are described in Brömme & Demuth (1994) Methods Enzymol. 244, 671-685. Peptide chloromethyl ketones as inhibitors of cysteine proteases are described in Williams & Mann (1993) Methods Enzymol. 222, 503-513. Thus, methods for synthesising suitable inhibitors are well known to the person skilled in the art.

It is preferred if the inhibitor is a chloromethylketone inhibitor or a fluoromethylketone inhibitor. It is less preferred if the inhibitor is a vinyl sulphone inhibitor. Chen et al (1998) FEBS Letters 441, 361-365 shows that legumain (AEP) has an active site similar to that of the proteases caspases, clostripain and gingipains. Caspases are aspartate-specific proteases whereas chlostripain and gingipains are arginyl and lysinyl proteases. AEP inhibitors may be made by modifying caspase inhibitors by replacing aspartate with asparagine, and they may be made by replacing arginine or lysine with asparagine in clostripain or gingipain inhibitors.

It is possible that, by analogy with other cysteine proteases, natural inhibitors of AEP exist, for example peptide inhibitors found in mammalian cells, or inhibitors found in microorganisms. The inhibitors for use in the invention include these inhibitors.

The AEP inhibitor is selective for AEP. By "selective" we mean that the inhibitor is at least ten times more potent for AEP than for another cysteine protease that does not have asparaginyl endopeptidase activity.

More preferably, the AEP inhibitor is at least 100 times more potent for AEP than for another cysteine protease inhibitor, still more preferably at least 1000 times more potent and most preferably at least 10 000 times more potent. In particular, it is preferred if the AEP inhibitor is an inhibitor which does not inhibit substantially cathepsins S and L. Cathepsins S and L are particular about residues which occupy the P2 position and inhibitors which contain aspartate or glutamate residues in the P2 position (ie the residue N-terminal of asparagine) are suitable for producing AEP inhibitors but are unfavourable for inhibitors of cathepsins L, S and B. Cathepsins S and L have a preference for uncharged amino acids in the P2 position (see Lysosomal Cysteine Proteases, Kirschke, Barrett & Rawlings, Second Edition, 1998, Oxford University Press, Oxford). Preferred inhibitors of AEP are those which include Ala-Glu-AsnQ where Q is defined as above. Particularly preferred inhibitors are those which include Ala-Glu-Asn-chloromethylketone or Ala-Glu-Asn-fluoromethylketone.

In a further preferred embodiment, the inhibitor of AEP may be an AEP-selective antisense agent.

Antisense agents are typically antisense oligonucleotides.

Antisense oligonucleotides are single-stranded nucleic acid, which can specifically bind to a complementary nucleic acid sequence. By binding to the appropriate target sequence, an RNA-RNA, a DNA-DNA, or RNA-DNA duplex is formed. These nucleic acids are often termed "antisense" because they are complementary to the sense or coding strand of the gene.

Recently, formation of a triple helix has proven possible where the oligonucleotide is bound to a DNA duplex. It was found that oligonucleotides could recognise sequences in the major groove of the DNA double helix. A triple helix was formed thereby. This suggests that it is possible to synthesise a sequence-specific molecules which specifically bind double-stranded DNA via recognition of major groove hydrogen binding sites.

By binding to the target nucleic acid, the above oligonucleotides can inhibit the function of the target nucleic acid. This could, for example, be a result of blocking the transcription, processing, poly(A)addition, replication, translation, or promoting inhibitory mechanisms of the cells, such as promoting RNA degradations.

Antisense oligonucleotides are prepared in the laboratory and then introduced into cells, for example by microinjection or uptake from the cell culture medium into the cells, or they are expressed in cells after transfection with plasmids or retroviruses or other vectors carrying an antisense gene. Antisense oligonucleotides were first discovered to inhibit viral replication or expression in cell culture for Rous sarcoma virus, vesicular stomatitis virus, herpes simplex virus type 1, simian virus and influenza virus. Since then, inhibition of mRNA translation by antisense oligonucleotides has been studied extensively in cell-free systems including rabbit reticulocyte lysates and wheat germ extracts. Inhibition of viral function by antisense oligonucleotides has been demonstrated *in vitro* using oligonucleotides which were complementary to the AIDS HIV retrovirus RNA (Goodchild, J. 1988 "Inhibition of Human Immunodeficiency Virus Replication by Antisense Oligodeoxynucleotides", Proc. Natl. Acad. Sci. (USA) 85(15), 5507-11). The Goodchild study showed that oligonucleotides that were most effective were complementary to the poly(A) signal; also effective were those targeted at the 5' end of the RNA, particularly the cap and 5' untranslated region, next to the primer binding site and at the primer binding site. The cap, 5' untranslated region, and poly(A) signal lie within the sequence repeated at the ends of retrovirus RNA (R region) and the oligonucleotides complementary to these may bind twice to the RNA.

Oligonucleotides are subject to being degraded or inactivated by cellular endogenous nucleases. To counter this problem, it is possible to use modified oligonucleotides, eg having altered internucleotide linkages, in which the naturally occurring phosphodiester linkages have been replaced with another linkage. For example, Agrawal et al (1988) Proc. Natl. Acad. Sci. USA 85, 7079-7083 showed increased inhibition in tissue culture of HIV-1 using oligonucleotide phosphoramidates and phosphorothioates. Sarin et al (1988) Proc. Natl. Acad. Sci. USA 85, 74.48-7451 demonstrated increased inhibition of HIV-1 using oligonucleotide methylphosphonates. Agrawal et al (1989) Proc. Natl. Acad. Sci. USA 86, 7790-7794 showed inhibition of HIV-1 replication in both early-infected and chronically infected cell cultures, using nucleotide sequence-specific oligonucleotide phosphorothioates. Leither et al (1990) Proc. Natl. Acad. Sci. USA 87, 3430-3434 report inhibition in tissue culture of influenza virus replication by oligonucleotide phosphorothioates.

Oligonucleotides having artificial linkages have been shown to be resistant to degradation *in vivo.* For example, Shaw et al (1991) in Nucleic Acids Res. 19, 747-750, report that otherwise unmodified oligonucleotides become more resistant to nucleases *in vivo* when they are blocked at the 3' end by certain capping structures and that uncapped oligonucleotide phosphorothioates are not degraded *in vivo.*

A detailed description of the H-phosphonate approach to synthesizing oligonucleoside phosphorothioates is provided in Agrawal and Tang (1990) Tetrahedron Letters 31, 7541-7544, the teachings of which are hereby incorporated herein by reference. Syntheses of oligonucleoside methylphosphonates, phosphorodithioates, phosphoramidates, phosphate esters, bridged phosphoramidates and bridge phosphorothioates are known in the art. See, for example, Agrawal and Goodchild (1987) Tetrahedron Letters 28, 3539; Nielsen et al (1988) Tetrahedron Letters 29, 2911; Jager et al (1988) Biochemistry 27, 7237; Uznanski et al (1987) Tetrahedron Letters 28, 3401 Bannwarth (1988) Helv. Chim. Acta. 71, 1517; Crosstick and Vyle (1989) Tetrahedron Letters 30, 4693; Agrawal et al (1990) Proc. Natl. Acad. Sci. USA 87, 1401-1405, the teachings of which are incorporated herein by reference. Other methods for synthesis or production also are possible. In a preferred embodiment the oligonucleotide is a deoxyribonucleic acid (DNA), although ribonucleic acid (RNA) sequences may also be synthesized and applied.

The oligonucleotides useful in the invention preferably are designed to resist degradation by endogenous nucleolytic enzymes. *In vivo* degradation of oligonucleotides produces oligonucleotide breakdown products of reduced length. Such breakdown products are more likely to engage in non-specific hybridization and are less likely to be effective, relative to their full-length counterparts. Thus, it is desirable to use oligonucleotides that are resistant to degradation in the body and which are able to reach the targeted cells.

The present oligonucleotides can be rendered more resistant to degradation *in vivo* by substituting one or more internal artificial internucleotide linkages for the native phosphodiester linkages, for example, by replacing phosphate with sulphur in the linkage. Examples of linkages that may be used include phosphorothioates, methylphosphonates, sulphone, sulphate, ketyl, phosphorodithioates, various phosphoramidates, phosphate esters, bridged phosphorothioates and bridged phosphoramidates. Such examples are illustrative, rather than limiting, since other internucleotide linkages are known in the art. See, for example, Cohen, (1990) Trends in Biotechnology*.* The synthesis of oligonucleotides having one or more of these linkages substituted for the phosphodiester internucleotide linkages is well known in the art, including synthetic pathways for producing oligonucleotides having mixed internucleotide linkages.

Oligonucleotides can be made resistant to extension by endogenous enzymes by "capping" or incorporating similar groups on the 5' or 3' terminal nucleotides. A reagent for capping is commercially available as AminoLink II^{™} from Applied BioSystems Inc, Foster City, CA. Methods for capping are described, for example, by Shaw et al (1991) Nucleic Acids Res. 19, 747-750 and Agrawal et al (1991) Proc. Natl. Acad. Sci. USA 88(17), 7595-7599, the teachings of which are hereby incorporated herein by reference.

A further method of making oligonucleotides resistant to nuclease attack is for them to be "self-stabilized" as described by Tang et al (1993) Nucl. Acids Res. 21, 2729-2735 incorporated herein by reference. Self-stabilized oligonucleotides have hairpin loop structures at their 3' ends, and show increased resistance to degradation by snake venom phosphodiesterase, DNA polymerase I and fetal bovine serum. The self stabilized region of the oligonucleotide does not interfere in hybridization with complementary nucleic acids, and pharmacokinetic and stability studies in mice have shown increased *in vivo* persistence of self-stabilized oligonucleotides with respect to their linear counterparts.

In accordance with the invention, the inherent binding specificity of antisense oligonucleotides characteristic of base pairing is enhanced by limiting the availability of the antisense compound to its intend locus *in vivo*, permitting lower dosages to be used and minimizing systemic effects. Thus, oligonucleotides are applied locally to achieve the desired effect. The concentration of the oligonucleotides at the desired locus is much higher than if the oligonucleotides were administered systemically, and the therapeutic effect can be achieved using a significantly lower total amount. The local high concentration of oligonucleotides enhances penetration of the targeted cells and effectively blocks translation of the target nucleic acid sequences.

The oligonucleotides can be delivered to the locus by any means appropriate for localized administration of a drug. For example, a solution of the oligonucleotides can be injected directly to the site or can be delivered by infusion using an infusion pump. The oligonucleotides also can be incorporated into an implantable device which when placed at the desired site, permits the oligonucleotides to be released into the surrounding locus.

The oligonucleotides may be administered *via* a hydrogel material. The hydrogel is noninflammatory and biodegradable. Many such materials now are known, including those made from natural and synthetic polymers. In a preferred embodiment, the method exploits a hydrogel which is liquid below body temperature but gels to form a shape-retaining semisolid hydrogel at or near body temperature. Preferred hydrogel are polymers of ethylene oxide-propylene oxide repeating units. The properties of the polymer are dependent on the molecular weight of the polymer and the relative percentage of polyethylene oxide and polypropylene oxide in the polymer. Preferred hydrogels contain from about 10 to about 80 % by weight ethylene oxide and from about 20 to about 90 % by weight propylene oxide. A particularly preferred hydrogel contains about 70 % polyethylene oxide and 30% polypropylene oxide. Hydrogels which can be used are available, for example, from BASF Corp., Parsippany, NJ, under the tradename Pluronic^{R}.

In this embodiment, the hydrogel is cooled to a liquid state and the oligonucleotides are admixed into the liquid to a concentration of about 1 mg oligonucleotide per gram of hydrogel. The resulting mixture then is applied onto the surface to be treated, for example by spraying or painting during surgery or using a catheter or endoscopic procedures. As the polymer warms, it solidifies to form a gel, and the oligonucleotides diffuse out of the gel into the surrounding cells over a period of time defined by the exact composition of the gel.

The oligonucleotides can be administered by means of other implants that are commercially available or described in the scientific literature, including liposomes, microcapsules and implantable devices. For example, implants made of biodegradable materials such as polyanhydrides, polyorthoesters, polylactic acid and polyglycolic acid and copolymers thereof, collagen, and protein polymers, or non-biodegradable materials such as ethylenevinyl acetate (EVAc), polyvinyl acetate, ethylene vinyl alcohol, and derivatives thereof can be used to locally deliver the oligonucleotides. The oligonucleotides can be incorporated into the material as it is polymerized or solidified, using melt or solvent evaporation techniques, or mechanically mixed with the material. In one embodiment, the oligonucleotides are mixed into or applied onto coatings for implantable devices such as dextran coated silica beads, stents, or catheters.

The dose of oligonucleotides is dependent on the size of the oligonucleotides and the purpose for which is it administered. In general, the range is calculated based on the surface area of tissue to be treated. The effective dose of oligonucleotide is somewhat dependent on the length and chemical composition of the oligonucleotide but is generally in the range of about 30 to 3000 µg per square centimetre of tissue surface area.

The oligonucleotides may be administered to the patient systemically for both therapeutic and prophylactic purposes. The oligonucleotides may be administered by any effective method, for example, parenterally (eg intravenously, subcutaneously, intramuscularly) or by oral, nasal or other means which permit the oligonucleotides to access and circulate in the patient's bloodstream. Oligonucleotides administered systemically preferably are given in addition to locally administered oligonucleotides, but also have utility in the absence of local administration. A dosage in the range of from about 0.1 to about 10 grams per administration to an adult human generally will be effective for this purpose.

AEP-selective antisense agents, such as antisense oligonucleotides, may be designed and made by the person skilled in the art by reference, for example, to the cDNA encoding AEP which is described in Chen et al (1997) J. Biol. Chem. 272, 8090-8098, incorporated herein by reference. It will be appreciated that there may be more than one AEP gene or that an AEP gene can give rise to more than one AEP transcript. Typically, the antisense oligonucleotide has at least 10 bases and, more preferably, at least 15 bases. Typically, the antisense oligonucleotides has around 18 to 20 bases. Although oligonucleotides may be designed and made which are complementary to any region of an AEP gene or mRNA transcript, in preferred embodiments the antisense oligonucleotides correspond to the region of the mRNA which contains the translation initiation signals or which encodes the N-terminus of AEP polypeptide. In addition, it is preferred if the sites in the AEP mRNA which are targeted by the antisense oligonucleotides are sites in which mRNA secondary structure is not expected and at which proteins are not expected to bind.

The AEP-inhibiting antisense agents need not be perfectly complementary to an AEP gene or mRNA in order to be effective. Rather, some degree of mismatches may be acceptable if the antisense oligonucleotide is of sufficient length. The oligonucleotides should have sufficient length and complementarity so as to selectively hybridise to an AEP transcript under physiological conditions. Preferably, mismatches are absent or minimal.

The AEP inhibitor, suitably, following administration to the patient, is able to contact and inhibit AEP at a suitable site in the patient. Although it is desirable for the AEP inhibitor to be cell permeant, it is believed that non-permeant AEP inhibitors may be useful since they may be taken into an appropriate cell by endocytosis.

An "effective amount" of the AEP inhibitor is an amount effective to modulate the immune response in the patient to a clinically useful extent.

AEP is believed to be involved in processing of protein antigens which are destined to be loaded into and presented by Class II MHC molecules. In a preferred embodiment of the invention the patient to be treated has or is at risk of a disease which involves MHC Class II molecules. Although we are not bound by any theory concerning the invention, we believe that AEP may play an important role in autoimmune disease because it may recognise and cleave at sites which are normally hidden by glycosylation at asparagine residues. Glycosylation at asparagine residues is common in mammalian proteins and glycosylated asparagine residues are not cleaved by AEP. An abnormal reduction in asparagine glycosylation of a self protein may lead to it being susceptible to AEP cleavage at these cryptic sites and, therefore, the peptide produced from the abnormally glycosylated protein may be susceptible to loading into and presentation to the immune system by Class II MHC molecules. Bacterial proteins, such as tetanus toxin, are typically not asparagine glycosylated and so, if they contain appropriate asparagine residues, are susceptible to degradation by AEP and presentation on Class II MHC molecules in any case.

Class II MHC molecules that remain associated with invariant chain cannot bind other peptides. Therefore, inhibition of AEP may reduce the competency of class II MHC molecules for binding antigenic peptides and reduces the presentation of antigenic peptides by Class II MHC molecules.

Preferably, the diseases are autoimmune diseases and it is particularly preferred if the patient to be treated has or is at risk of an autoimmune disease such as rheumatoid arthritis, insulin-dependent diabetes mellitus, multiple sclerosis, Hashimoto's thyroiditis, coeliac disease, myasthenia gravis, pemphigus vulgaris, systemic lupus erythromatosus and Grave's disease.

In further preferred embodiments of the invention the patient in need of modulation of the immune system has or is at risk of an allergic reaction or has undergone, or is to undergo, an organ or tissue or cell transplant in which graft rejection may ensue.

Several allergies and hypersensitivity reactions involve undesired responses to harmless environmental antigens inhaled, ingested or otherwise contacted. In several cases the antigens involved are known and, in as much as CD4 T cells are activated by class II MHC/peptide complexes, inhibition of processing of these antigens by AEP inhibitors is believed to be likely to block or reduce their display to the T cells which cause the allergic or hypersensitive reactions.

### Typical allergies include:

Allergic rhinitis (hay fever) and allergic asthma induced by grass and tree pollens, dust-mite faeces, cat/dog fur and so on; contact hypersensitivity reactions induced by: metals (nickel, cobalt, copper, palladium), pentadecacatechol (poison ivy) or bee venom; food allergies to shellfish, milk, eggs, fish, wheat; and drug allergies eg to penicillin, aspirin.

Thus, the AEP inhibitors of the invention are useful in the manufacture of medicaments for treating allergies and allergic reactions and hypersensitivity reactions. In addition to those allergies and hypersensitivity reactions mentioned above, it is contemplated that the AEP inhibitors described herein are useful in the manufacture of medicaments for treating allergic or hypersensitivity conditions associated with any of the following: the feline skin and salivary gland allergen of the domestic cat *Felis domesticus* (the amino acid sequence of which is disclosed in WO 91/06571), the major protein allergens from the house dust mite dermatophagoides (amino acid sequences disclosed in WO 94/24281) and allergens present in any of the following: grass, tree and weed (including ragweed) pollens; fungi and molds; foods eg fish, shellfish, crab, lobster, peanuts, nuts, egg and milk; stinging insects eg bee, wasp and hornet and the chironomidae (non-biting midges); spiders and mites; mammals such as dog, horse, rat, guinea pig, mice and gerbil; latex; biological detergent additives; drugs eg penicillins and other antibiotics and anaesthetic agents.

More particularly allergies to insect proteins arise from proteins from the housefly, fruit fly, sheep blow fly, screw worm fly, grain weevel, silkworm, honeybee, non-biting midge larvae, bee moth larvae, mealworm, cockroach and larvae of *Tenibrio molitor* beetle. All of these insect allergens are of particular relevance to allergic problems arising in the workplace.

It will be appreciated that the AEP inhibitors may be used on their own, or that they may be prepared for administration in conjunction with other forms of treatment of the allergic or hypersensitivity conditions, which are known in the art.

Allografts (from same species, different individual) or xenografts (different species) are frequently rejected because of differences at the MHC genetic locus between the donor and the recipient. MHC matching improves the chances of graft acceptance as do immunosuppressive drug regimes. Blockade or reduction in the presentation of peptides on class II MHC molecules using the inhibitors of AEP described herein may be useful in immunosuppression and may be used instead of or in conjunction with existing suppressors of T cell activation such as cyclosporin or FK506.

Tissue and organ transplants which may benefit from additional immunosupressive methods include kidney, lung, heart, liver, cornea and bone marrow. Prior treatment of both the organ and host may be beneficial.

It will be appreciated that the AEP inhibitors, may be used on their own, or that they may be prepared for administration in conjunction with other forms of immunosuppression such as those described above or as mixtures of AEP inhibitors or as mixtures of an AEP inhibitor with, for example, an inhibitor of cathepsin S as described below.

The aforementioned inhibitors for use in the invention or a formulation thereof may be prepared for administration by any conventional method including oral, parenteral (eg subcutaneous or intramuscular) injection topical and the like. The treatment may consist of a single dose or a plurality of doses over a period of time.

Whilst it is possible for an inhibitor for use in the invention to be prepared for administration alone, it is preferable to present it as a pharmaceutical formulation, together with one or more acceptable carriers. The carrier(s) must be "acceptable" in the sense of being compatible with the compound of the invention and not deleterious to the recipients thereof. Typically, the carriers will be water or saline which will be sterile and pyrogen free.

The formulations may conveniently be presented in unit dosage form and may be prepared by any of the methods well known in the art of pharmacy. Such methods include the step of bringing into association the active ingredient (inhibitor of AEP) with the carrier which constitutes one or more accessory ingredients. In general the formulations are prepared by uniformly and intimately bringing into association the active ingredient with liquid carriers or finely divided solid carriers or both, and then, if necessary, shaping the product.

Formulations in accordance with the present invention suitable for oral administration may be presented as discrete units such as capsules, cachets or tablets, each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a binder (eg povidone, gelatin, hydroxypropylmethyl cellulose), lubricant, inert diluent, preservative, disintegrant (eg sodium starch glycolate, cross-linked povidone, cross-linked sodium carboxymethyl cellulose), surface-active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein using, for example, hydroxypropylmethylcellulose in varying proportions to provide desired release profile.

Formulations suitable for topical administration in the mouth include lozenges comprising the active ingredient in a flavoured basis, usually sucrose and acacia or tragacanth; pastilles comprising the active ingredient in an inert basis such as gelatin and glycerin, or sucrose and acacia; and mouth-washes comprising the active ingredient in a suitable liquid carrier.

Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilised) condition requiring only the addition of the sterile liquid carrier, for example water for injections, immediately prior to use. Extemporaneous injection solutions and suspensions may be prepared from sterile powders, granules and tablets of the kind previously described.

Preferred unit dosage formulations are those containing a daily dose or unit, daily sub-dose or an appropriate fraction thereof, of an active ingredient.

It should be understood that in addition to the ingredients particularly mentioned above the formulations of this invention may include other agents conventional in the art having regard to the type of formulation in question, for example those suitable for oral administration may include flavouring agents.

It will be appreciated that the pharmaceutical composition for use in the invention may further comprise an agent which is usefully administered to the patient in need of modulation of their immune response. For example, the composition may further comprise an agent for treatment or prevention or amelioration of an autoimmune disease or allergic or hypersensitivity reaction. As a further example, the composition may further comprise an immunosuppressive agent such as cyclosporin or FK506.

It is particularly preferred if the AEP inhibitor is for administration at or near to the site of the disease. For example, in the treatment of rheumatoid arthritis it is desirable for the AEP inhibitor to be for administration at or close, to the joints affected. In this case, the AEP inhibitor may be for administration by topical application.

In addition to AEP (as described for the first time herein), two other classes of proteases are involved in the Class II MHC pathway. These are the papain family of cysteine proteases (cathepsins S, L, B and H), and the aspartic acid family (cathepsins D and E).

Recent results from cathepsin L, D and B knock-out mice suggest that they have no major effect, at least in peripheral antigen presenting cells. This suggests that either there is considerable redundancy or that these enzymes are not the really important enzymes. The results described herein show that AEP is an important enzyme in processing Class II MHC antigens, and it is contemplated that the inhibitors of AEP when used alone may be useful. However, in some circumstances it may be desirable to administer to the patient, or combine in a pharmaceutical formulation or in a therapeutic system (kit of parts), an inhibitor of AEP with an inhibitor of one or more of these other enzymes which are involved in the Class II MHC pathway. In particular, since cathepsin S appears to be an important enzyme in invariant chain processing (see, for example, WO 97/40066) it is believed that a combination of cathepsin S inhibitors and AEP inhibitors may be particularly useful.

Thus, combinations of AEP inhibitors as disclosed herein and inhibitors cathepsin D or cathepsin E may be used. Known inhibitors of aspartic acid enzymes such as cathepsin D and cathepsin E include, for example, pepstatin and CGP 53437 (Ciba-Geigy; see Alteri et al (1993) Antimic. Ag. Chemotherapy 37, 2087-).

Similarly, combinations of AEP inhibitors and lysosomal cysteine protease inhibitors (ie inhibitors of cathepsin S, L, B and H) are contemplated. Broad specificity inhibitors of these enzymes include leupeptin and the epoxide compound E64 or E64D. However, it is contemplated that cathepsin S inhibitors (in particular cathepsin S-selective or specific inhibitors) may be particularly advantageously combined with AEP inhibitors. Cathepsin S inhibitors are described in detail in WO 97/40066, all of which are incorporated herein by reference. In particular, the following cathepsin S inhibitors are believed to be useful in combination with AEP inhibitors: morpholino-Leu-homophenylalanine-vinyl sulphone; Leu-Leu-Leu-vinyl sulphone with various N-terminal blocking groups; vinyl sulphones based on the invariant chain cleavage sites cut by cathepsin S.

It is preferred that combinations of invariant chain-based inhibitors targeted to both AEP and cathepsin S are used. Combinations of such inhibitors may readily be devised by the skilled person by reference to the AEP inhibitors disclosed herein and by reference to the invariant chain-based cathepsin S inhibitors described in WO 97/40066. Examples of such combinations include, but are not limited to, Bl-Glu-Asn-Leu-vinyl sulphone and/or Bl-Leu-Arg-Met-vinyl sulphone (to block cathepsin S) plus one or more of Bl-Ser-Gln-Asn; Bl-Leu-Glu-Asn; Bl-Leu-Gln-Asn; BI-Pro-Glu-Asn; and Bl-Leu-Lys-Asn, all as vinyl sulphones (attached via the Asn residue).

As an alternative to vinyl sulphones as the reactive group, any of the reactive groups mentioned above ("Q") may be used in the inhibitors.

Other suitable combinations include invariant chain sequence based competitive inhibitors (as disclosed herein for AEP and as disclosed in WO 97/40066 for cathepsin S); and compounds disclosed herein as AEP inhibitors which are not based on the invariant chain in combination with any cathepsin S inhibitor.

The invention also includes an in vitro method of reducing the processing of a protein antigen, or reducing presentation of a protein antigen by a MHC Class II molecule, by a cell, the method comprising contacting the cell with an inhibitor of asparaginyl endopeptidase.

In one embodiment of the invention, an appropriate antigen presenting cell (APC), such as a dendritic cell or macrophage or B lymphocyte is transfected with a pool of cDNAs (or is contacted with Proteins expressed from a pool of cDNAs) wherein the pool of cDNAs is believed to contain a cDNA encoding a protein which activates a T cell and may be associated with an autoimmune disease. The cDNA which encodes a protein which activates a T cell is selected and encodes a putative autoimmune protein. The assay may be carried out in the presence or absence of AEP inhibition in order to determine the involvement of AEP processing in the disease and also to determine any cryptic Asn glycosylation sites which may be important in the self-antigen immune response. Thus, the T cell is used to "read out" whether the cDNA for a putative autoantigen is being expressed and being presented, and whether AEP inhibitors have any effect on this.

A further aspect of the invention provides a method of identifying a compound for modulating Class II MHC antigen processing the method comprising contacting a test compound with asparaginyl endopeptidase and selecting a compound which reduces its activity, and determining whether the so selected compound is capable of substantially inhibiting the loading and presentation of peptides on an appropriate Class II MHC molecule-containing cell.

This method (or screening assay) of the invention is suitable carried out in a format which allows for many test compounds to be screened simultaneously such as in a 96-well plate format. The activity of AEP is conveniently measured using any suitable substrate but it is preferred if the substrate is one which, upon cleavage by AEP, gives rise to a readily-detectable product. For example, the product may be coloured or fluorescent or detectable in some other way. It is particularly preferred if the product is fluorescent; a preferred substrate is Z-Ala-Ala-Asn 7-(4-methyl)coumarylamide.

The test compound is preferably a compound from a library of test compounds. The compound may be one which has been made by synthetic chemistry methods (such as combinatorial chemistry methods) or it may be a naturally occurring compound. Without prejudice to the type of compounds which can usefully be screened (which include any suitable compounds), it is believed that many inhibitors of AEP will have a peptide or peptide-like structure. Thus, it is preferred if the test compound has a peptide or peptide-like structure.

It will be appreciated that the method or screening assay may also include a step for eliminating non-specific inhibitory compounds or for positively selecting those inhibitors which inhibit AEP but do not substantially inhibit other cysteine proteases which do not have AEP activity.

A convenient way of determining whether the compound is capable of substantially inhibiting the loading and presentation of peptides on an appropriate Class II MHC molecule-containing cell is to assess whether the compound is capable of substantially inhibiting T cell activation by an appropriate Class II MHC molecule-containing cell. This may be done as described in the Example.

A further aspect of the invention comprises a non-human transgenic animal wherein a gene encoding asparaginyl endopeptidase has been modified and the animal expresses substantially no asparaginyl endopeptidase from said gene.

By "transgenic" animal we specifically include animals in which all or part of a gene have been "knocked out" or otherwise made substantially incapable of expressing an asparaginyl endopeptidase. Suitable mice can be made using standard methodology involving, for example, genetic manipulation of embryonic stem (ES) cells as is well known in the art. A cDNA encoding AEP is described in Chen et al (1997) J. Biol. Chem. 272, 8090-8096, and this information may be used in designing and making the transgenic animals of the invention.

It is particularly preferred if the non-human animal is a mouse, but it may be any suitable non-human animal including rat, rabbit and the like.

In a preferred embodiment of the invention, the non-human transgenic animal which expresses substantially no AEP from an AEP gene comprises a genetic background which predisposes the animal to an autoimmune disease either spontaneously or upon administration of protein antigen. Animals, especially mice, with a suitable genetic background are well known, for example NOD mice, which are a model for diabetes, EAE (experimental autoimmune encephalomyelitis) mice which are a model for allergic encephalomyelitis and CIA mice which are a model for collagen-induced arthritis. The mice used for the model disease EAE are of the MHC haplotypes H-2u or H-2k (eg PL/J or B10.PL strains). The mice used for the model disease CIA are of the MHC haplotype H-2q (eg DBA/1J strain). Such mice are described or referenced in Cantorna et al (1998) J. Nutr. 128, 68-72; Xiao & Link (1997) Clin. Immunol. Immunopathol. 85, 119-128; and Kumar et al (1997) J. Exp. Med. 185, 1725-1733.

Alternatively, the animal will be transgenic for a human Class II MHC molecule that predisposes to a human autoimmune disease.

In a further preferred embodiment, the non-human transgenic animal which expresses substantially no AEP from an AEP gene is further transgenic for a human Class II MHC molecule and, optionally, further transgenic for CD4. Mice with these further characteristics are described in Altmann et al (1995) J. Exp. Med. 181, 867-875; Yamamoto et al (1994) J. Ex. Med. 180, 165-171; and Woods et al (1994) J. Exp. Med 180, 173-181.

It will be appreciated that non-human animals which have combinations of characteristics can be made by crossing appropriate animals. Thus, the animals of the preferred embodiments of the invention may be made by crossing suitable animals.

From the foregoing it will be appreciated that new AEP inhibitors are disclosed.

A further aspect of the invention provides an inhibitor of asparaginyl endopeptidase which has the structure B1-(Xₐ-Xₙ)-Asn-Q wherein B1 is any suitable N terminal blocking group; Xₐ-Xₙ are the n amino acid residues immediately N terminal to an Asn cleavage site in the invariant chain of Class II MHC molecules; Asn is an asparagine residue; and Q is a group capable of reacting with the active site of asparaginyl endopeptidase.

The invention also includes its use in medicine.

Suitable blocking groups, amino acids, and groups capable of reacting with the active site of AEP are the same as disclosed earlier in the application. It is preferred if the group capable of reacting with the active site of AEP can react with the active site cysteine residue.

The n amino acid residues N terminal to an Asn cleavage site can readily be determined by reference to the sequence of the invariant chain and by reference to the Asn residue number as described earlier. Typically the number of amino acid residues is between 1 and 25; preferably, it is between 2 and 10; more preferably between 2 and 5.

Preferred inhibitors include. Bl-Ser-Gln-Asn-Q; Bl-Leu-Glu-Asn-Q; Bl-Leu-Gln-Asn-Q; Bl-Pro-Glu-Asn-Q; Bl-Leu-Lys-Asn-Q; Bl-Gln-Asn-Q; Bl-Glu-Asn-Q; Bl-Asp-Glu-Asn-Q; Bl-Asn-Gly-Asn-Q; Bl-Phe-Pro-Asn Q; Bl-Val-Pro-Asn-Q; and Bl-His-His-Asn-Q where Q is any reactive group as defined earlier and Bl is any N-terminal blocking group as defined earlier.

Further aspects provide an inhibitor according to Claims 38 and 40.

A still further aspect of the invention provides an inhibitor of asparaginyl endopeptidase which has the structure (Xb-Xc)Asn(Xd-Xe) wherein (Xb-Xc) are the r amino acid residues immediately N terminal to an Asn cleavage site in the invariant chain of Class II MHC molecules and (Xd-Xe) are the s amino acid residues immediately C terminal to an Asn cleavage site in the said invariant chain; Asn is an asparagine residue; and r and s are independently between 2 and 25, for use in medicine.

Preferably, r and s are independently between 2 and 10; more preferably r and s are independently between 2 and 5.

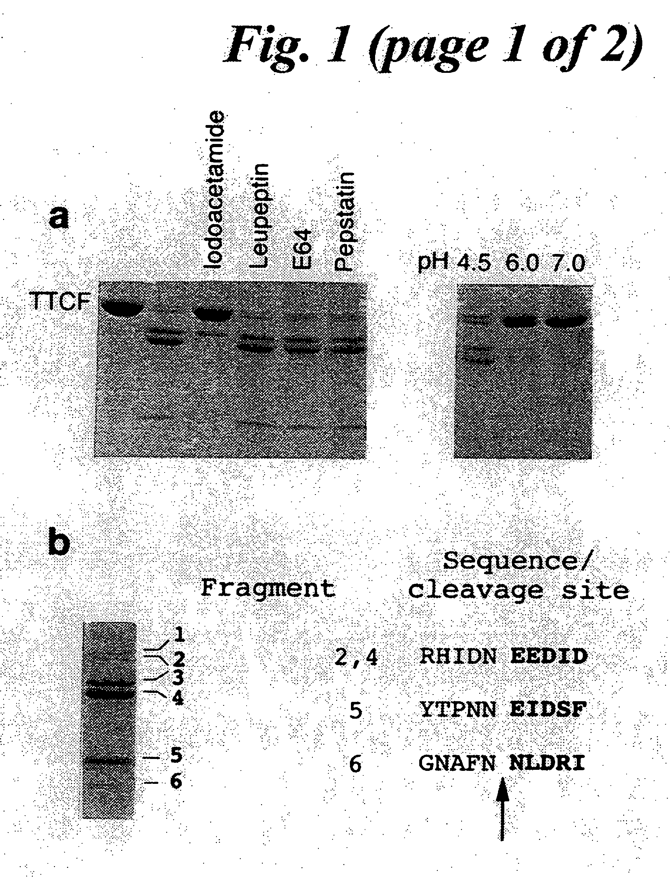

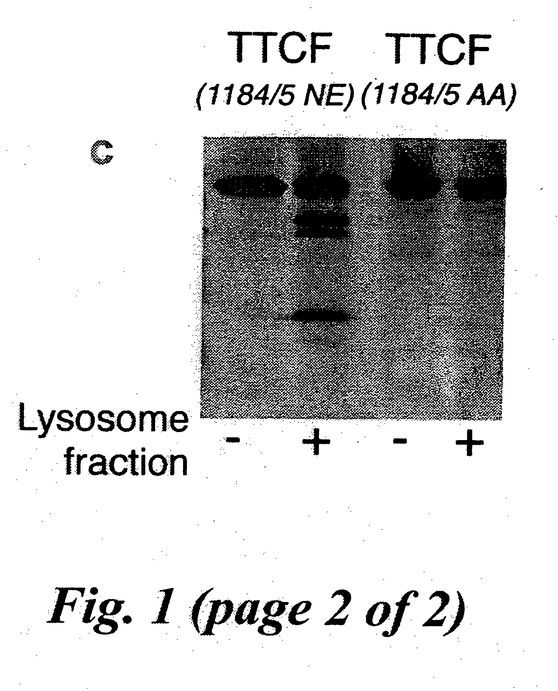

The invention will now be described by reference to the following Examples and Figures wherein
**Figure 1**: Processing of TTCF by a leupeptin-insensitive cysteine endopeptidase activity. (a) TTCF was digested *in vitro* in the presence of disrupted lysosomes (1-2 µg) in the presence or absence of iodoacetamide (1 mM), leupeptin, E64 or pepstatin (each at 0.1 mg/ml). Digestions were in 0.1 M NH₄ acetate at pH 4.5 or Na₂HPO₄, pH 6.0 or 7.0. After 4 hours at 37°C, the reactions were separated by Tris-tricine SDS-PAGE and stained with Coomassie Blue. (b) Cleavage sites: TTCF digestion products were separated by SDS PAGE and electrophoretically transferred to nitrocellulose membrane. Individual fragments numbered 1-6 were subjected to 5 cycles of Edman degradation. N-terminal sequence obtained is shown in bold following upstream residues from TTCF (tetanus toxin numbering). The N-terminal sequence of His-tagged TTCF was obtained for fragments 1 and 3. (c) Residues Asn 1184 and Glu 1184 in TTCF were mutated to alanine (see Methods) and digestions performed as before. The mutation abolishes cleavage at the mutated site but not at the first site (doublet at 47 kD). In this experiment cleavage of the third site was minimal.

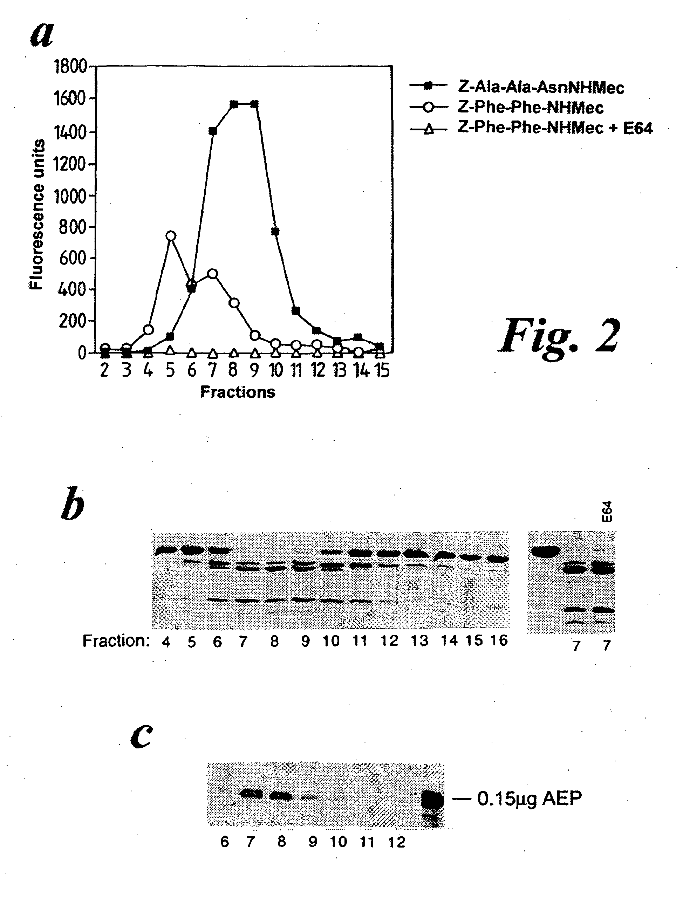

**Figure 2****:** TTCF is processed by an asparaginyl endopeptidase. (a) Chromatography of the TTCF processing activity on Mono-S resin. Fractions eluted by NaCl gradient were incubated with the substrates Z-Ala-Ala-Asn-NHMec or Z-Phe-Arg-NHMec in the presence or absence of E64 (trans-epoxysuccinyl-L-leucylamido-(4-guanidino)butane). E64 had no inhibitory effect on the cleavage of Z-Ala-Ala-Asn-NHMec (not shown). (b) Fractions as above were incubated with 10 µg TTCF in the presence or absence of E64 for 4 hours at 37°C prior to SDS-PAGE analysis. (c) Each fraction was analysed by SDS-PAGE and Western blotting. Electrophoretically transferred material was probed with an affinity purified antiserum raised against the peptide KGIGSGKVLKSGPQC from human legumain¹.

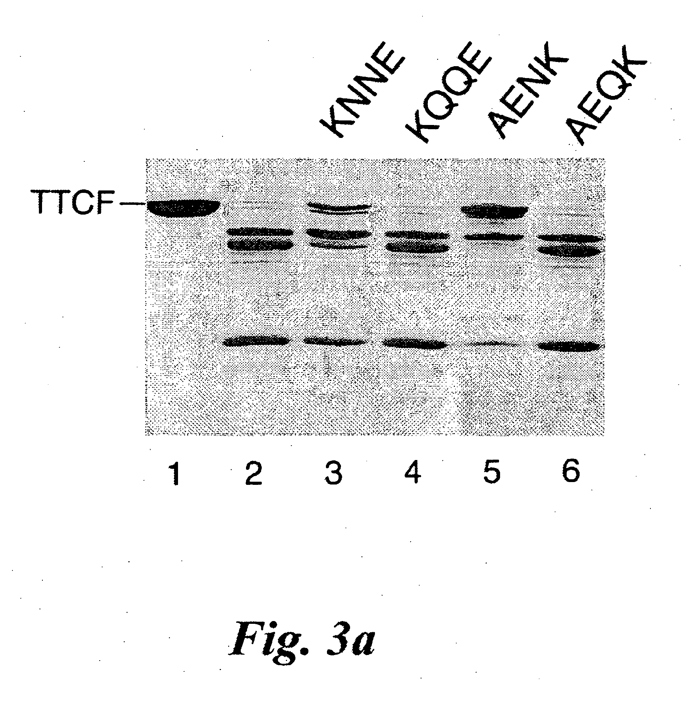

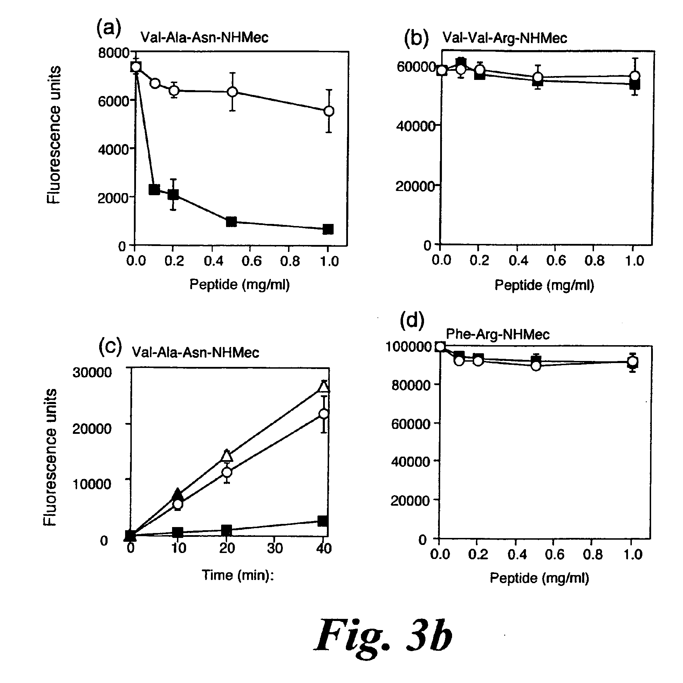

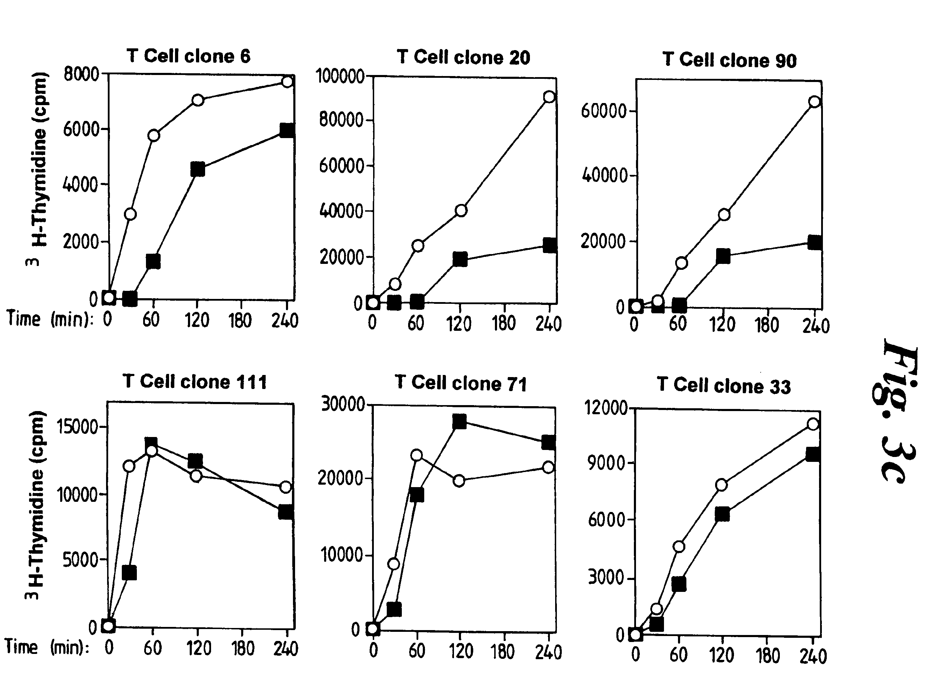

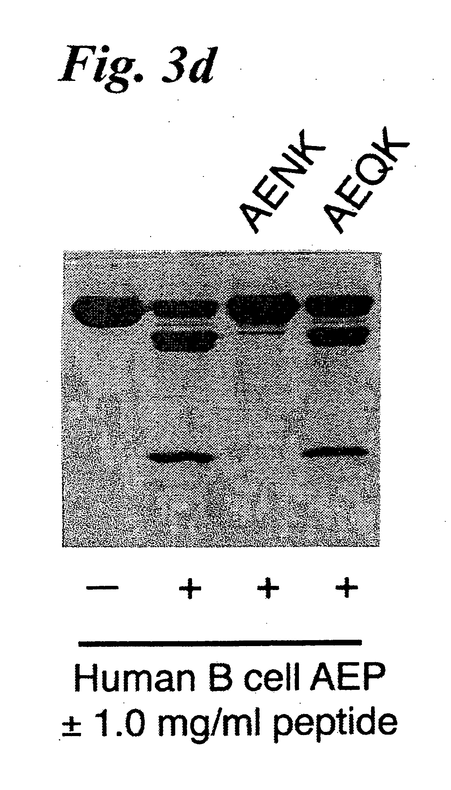

**Figure 3**: Peptide inhibitors of AEP block asparaginyl endopeptidase activity and TTCF processing *in vitro* and *in vivo.* (a) TTCF was digested with purified AEP (legumain from pig kidney) in the presence or absence of 0.2 mg/ml of the peptides indicated. Peptides were N and C-terminally blocked by not removing Fmoc or amide groups respectively. (b) Purified AEP or a crude lysosomal protease mixture were incubated with Z-Ala-Ala-Asn-NHMec, Z-Phe-Arg-NHMec (cathepsins L&B) or Z-Val-Val-Ala NHMec (cathepsin S) in the presence of increasing concentrations of AENK or AEQK. Release of 7-amino-4-methyl coumarin was measured after 10, 20 or 30 minutes. (c) Peripheral blood mononuclear cells were isolated from donor A.K. and preincubated for 15 minutes in the presence or absence of 1 mg/ml AENK (■) or AEQK (○) prior to addition of TTCF antigen. At different times the cells were washed, fixed in 0.05 % glutaraldehyde and co-cultured with autologous TTCF specific T cell clones. (d) Human B-cell AEP digests TTCF and is inhibited by AENK but not AEQK.
**Figure 4** shows the structure of various AEP inhibitors.

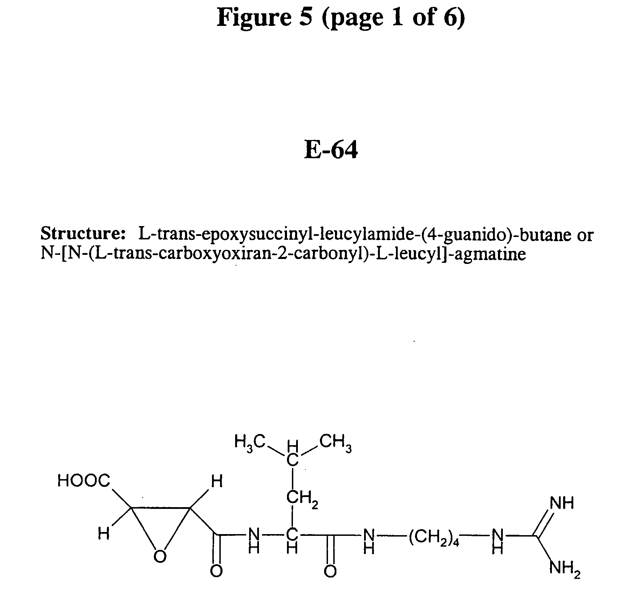

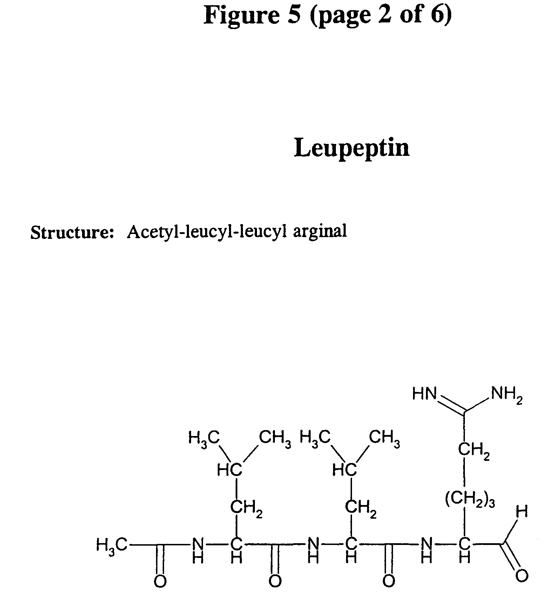

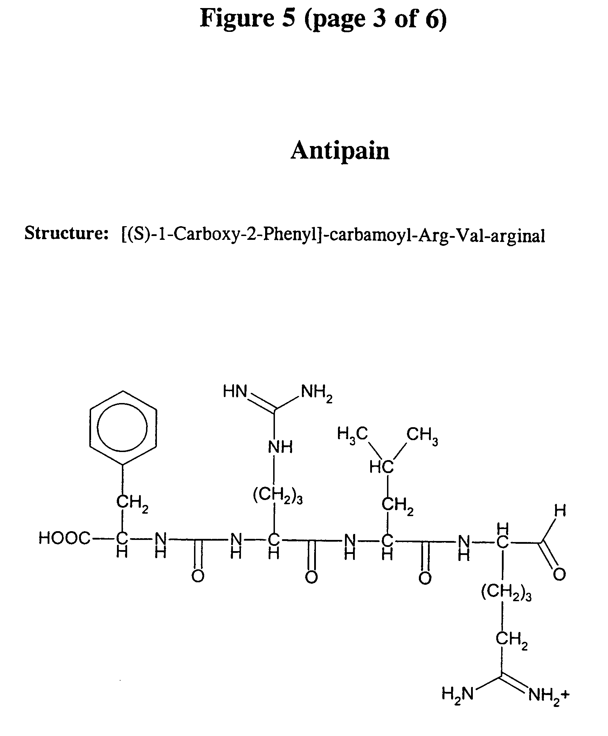

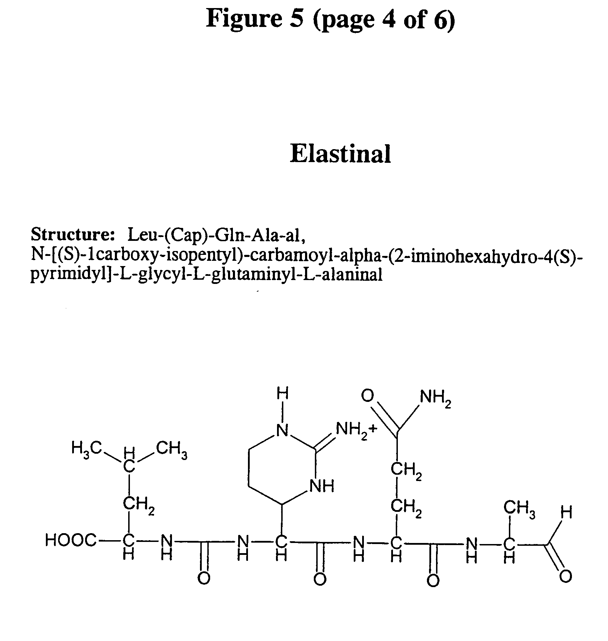

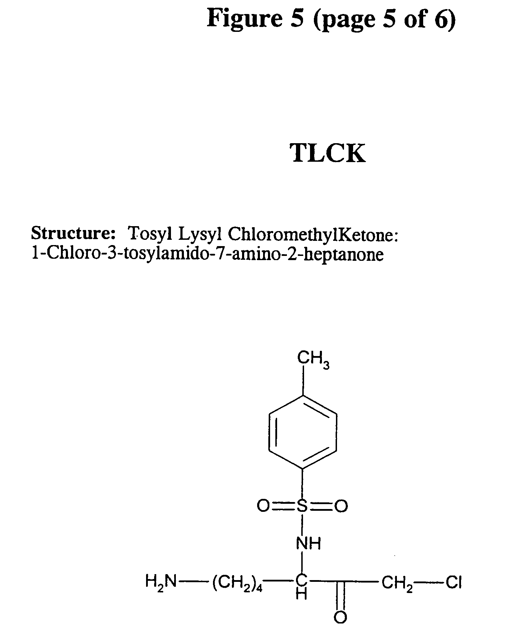

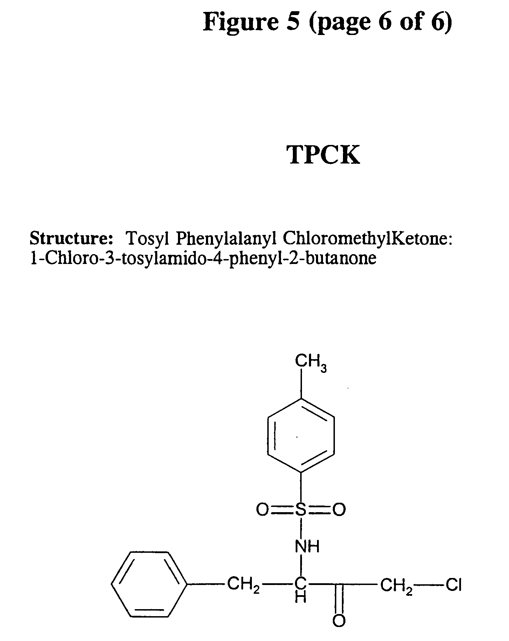

**Figure 5** shows the structure of various protease inhibitors which can be converted to AEP inhibitors as described in the text.

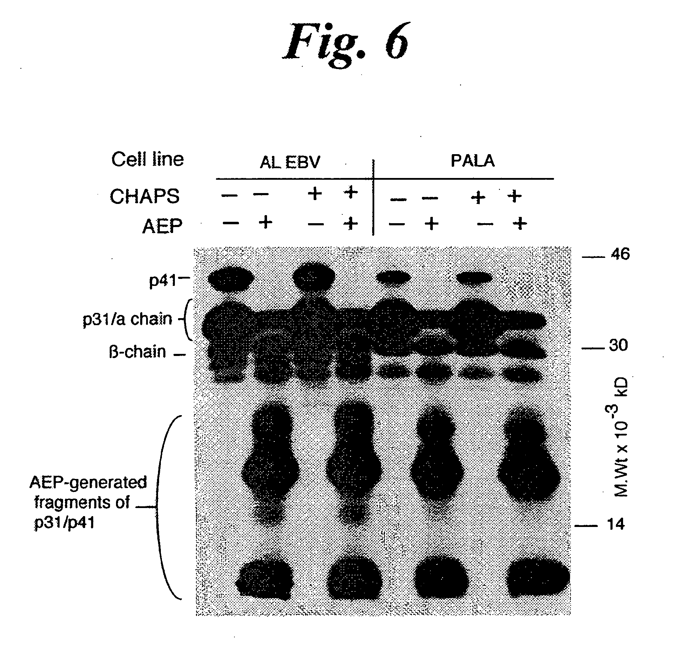

**Figure 6** shows that AEP can cleave the Class II MHC invariant chain (both p31 and p41 isoforms).
**Figure 7** shows further details of AEP processing of the invariant chain.
**Figure 8** shows the processing sites for AEP/legumain in diverse proteins. Proteins obtained from commercial sources, as described in the literature or custom peptides were digested with purified pig kidney legumain at pH 5.5 in the presence of 1 mM DTT. Digestion products were analysed by MALDI-TOF mass spectrometry and/or N-terminal Edman degradation following electrophoresis and electrophoretic transfer to PVDF membrane.

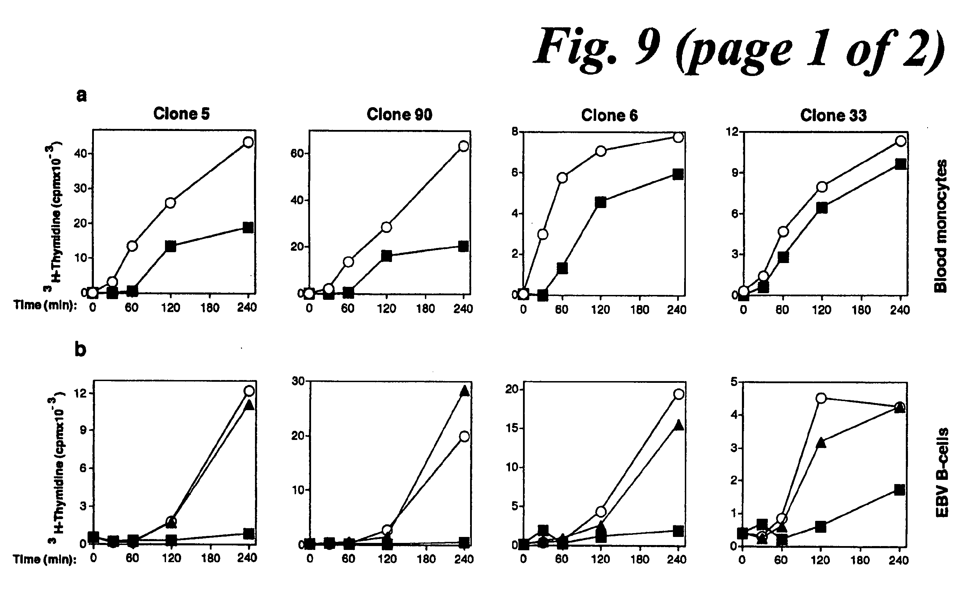

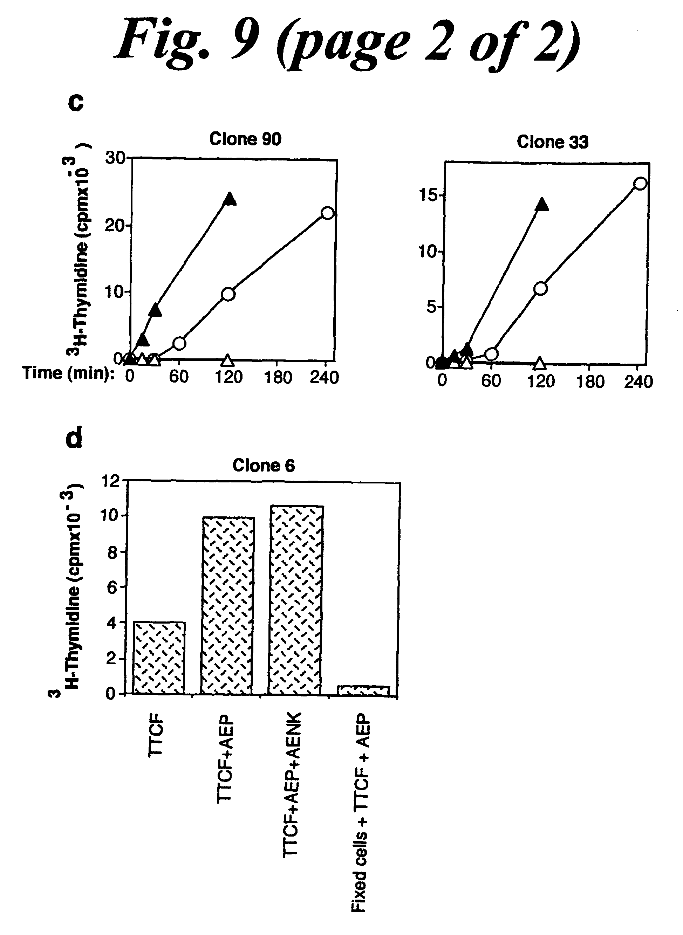

**Figure 9** shows that TTCF processing and presentation requires AEP *in vivo.* **a & b**, Peptide inhibitors of asparaginyl endopeptidase slow antigen presentation *in vivo.* **a**, Peripheral blood mononuclear cells or **b**, EBV-transformed B cells, both from donor A.K. were preincubated for 15 minutes in the presence of AENK (■), AEQK (○) or no peptide (▲), prior to addition of TTCF antigen. When present peptide was maintained during antigen pulsing at 1 mg/ml (**a**) or 2 mg/ml (**b**). At different times the cells were washed, fixed in 0.05 % glutaraldehyde and antigen presentation measured using proliferation of different autologous TTCF-specific T cell clones. **c**, AEP pre-processing of TTCF speeds antigen presentation. Antigen presentation was measured as in a, but using either TTCF (○) or TTCF pre-digested with AEP (▲, Δ) using either live (○, ▲) or fixed (Δ) autologous PBMC. **d**, Presentation of AEP-pre-processed antigen is no longer inhibited by AENK. Presentation was measured after 60 minutes of antigen pulsing using EBV B cells.

### Example 1: Asparaginyl endopeptidase is involved in processing and class II MHC presentation of a microbial antigen

### Summary

Foreign antigens must be proteolytically processed to allow loading of peptides onto class II MHC molecules. To investigate which proteases might be involved we exposed a domain of the microbial antigen, tetanus toxin (TTCF) to disrupted lysosomes purified from a human B cell line. Surprisingly, the dominant processing activity was not one of the known lysosomal cathepsins but rather an asparagine specific cysteine endopeptidase which we call AEP. This enzyme appears similar if not identical to a mammalian homologue of legumain¹, an asparaginyl endopeptidase found originally in plants and parasites^{2,3}. We designed competitive peptide inhibitors of AEP which specifically block its asparaginyl endopeptidase activity and inhibit processing of TTCF *in vitro* and its presentation *in vivo* to T cells. Since N-glycosylation renders asparagine resistant to cleavage by AEP we suggest that this enzyme may represent a further example of the ability of the innate immune system to focus its attention on microbial non-self.

To analyse processing of a foreign antigen without making prior assumptions about which enzymes are involved we exposed a 47 kD domain of the tetanus toxin antigen (TTCF) to disrupted lysosomes isolated from the human B cell line EDR. As shown in Fig 1a, the antigen was fragmented to produce a discrete series of products at pH 4.5 but not at pH 7.0 and minimally at pH 6.0, confirming the lysosomal origin of the protease(s) involved (Fig 1a). Surprisingly, we could not inhibit lysosomal digestion of TTCF with leupeptin or E64(trans-epoxysuccinyl-L-leucylamido-(4-guanidino)butane), broad spectrum inhibitors of lysosomal cysteine proteases or with pepstatin, an inhibitor of the aspartic acid cathepsins E and D (Fig 1a). LHVS, a more specific inhibitor of cathepsin S⁴ was also without effect (not shown). Nor could we reproduce the lysosomal TTCF digestion pattern or indeed generate any of these fragments using purified cathepsins L, S, B, D or E , all of which have been implicated in the class II MHC antigen processing pathway (reviewed in refs 5 & 6). Further inhibitor studies revealed however that the activity was sensitive to iodoacetamide (Fig 1a), to N-ethyl maleimide and to high concentrations of the diazomethane, Z-Phe-Phe-CHN2 (not shown) indicating that one or more cysteine protease(s) were nonetheless involved.

We sequenced each major TTCF digestion products to gain more information on this processing activity. All visible fragments arose from 3 cleavages in the TTCF protein and each occurred after an asparagine residue (Fig 1b). Mutation of asparagine 1184 and glutamic acid 1185 to alanine completely abolished cleavage at this site (Fig 1c) indicating that one (presumably the asparagine) or both of these residues is crucial for processing.

These results suggested that B cell lysosomes contain one or more novel cysteine endopeptidase(s) with possible specificity for asparagine. Although not previously described in antigen presenting cells, an enzyme with similar properties called legumain has been found in the seeds of legumimous plants^{2,3}, in *Schistosoma mansoni³* and recently in some mammalian tissues such as kidney and placental. We next partially purified the TTCF processing activity from a crude B cell lysosome fraction to establish whether or not it was an asparaginyl endopeptidase. As shown in Figure 2b, a peak of TTCF processing activity eluted from a cation-exchange resin at 0.4M NaCl. This activity co-eluted precisely with an activity capable of cleaving the substrate Z-Ala-Ala-Asn-7-(4-methyl)coumarylamide substrate (Fig 2a). Another protease activity, presumably cathepsin L and/or B, capable of cleaving the substrate Z-Phe-Arg-NHMec, partially overlapped the asparaginyl endopeptidase activity (Fig 2a). However this activity was completely inhibited by E64 (Fig 2a) while both the TTCF and Z-Ala-Ala-Asn-NHMec processing activity were unaffected (Fig 2b) confirming that a novel cysteine endopeptidase was involved. To compare this activity to the recently described mammalian form of legumain we generated anti-peptide antisera to several regions of the mammalian preprolegumain sequence¹. Blotting of each fraction with an antisera to residues 125-140 (see methods) revealed the presence of a protein with the same mobility (35 kD) as purified pig kidney legumain which co-eluted with the TTCF and peptide substrate processing activity (Fig 2c). Finally, exactly the same processing sites were recognised by the crude lysosomal fraction, the partially purified TTCF processing activity and purified pig kidney legumain (Figs 1b & 2b, ref 1 and data not shown). Thus, mammalian legumain or a closely related enzyme, is present in human B lymphocytes and *in vitro,* is the major enzyme responsible for processing this antigen. We propose the name AEP (Asparaginyl endopeptidase) for this enzyme to distinguish it from the plant enzyme.

We decided to test the possibility that high concentrations of asparagine containing peptides might act as competitive inhibitors. As shown in Fig 3, the N and C-terminally blocked tetrapeptide Ala-Glu-Asn-Lys-NH (AENK) substantially inhibited the processing of TTCF by AEP (Fig 3a). Lys-Asn-Asn-Glu-NH (KNNE) also inhibited but was less effective. Importantly, the same concentrations of the glutamine analogues AEQK and KQQE did not inhibit TTCFr processing demonstrating specific competitive inhibition of AEP (Fig 3a). To establish that inhibition of AEP was specific, we measured the hydrolysis of different synthetic peptide substrates in the presence and absence of these tetrapeptides. As shown in Fig 3b, AENK completely blocked cleavage of Z-Val-Ala-Asn-NHMec by B cell lysosomal fractions but had no effect on the hydrolysis of the cathepsin L/B substrate, Z-Phe-Arg-NHMec or on the Cathepsin S substrate Z-Val-Val-Arg-HNMec.

If AEP plays a role in TTCF processing in antigen presenting cells then sufficiently high levels of the competing AENK peptide might be expected to interfere with normal class II MHC loading of TTCF epitopes. Following a 15 minute preincubation with or without tetrapeptides, freshly isolated peripheral blood mononuclear cells (PBMC) were incubated with TTCF antigen in the continued presence or absence of the peptides. At different times, the cells were washed, fixed and then co-cultured with different autologous T cell clones to assess expression of different peptide/MHC complexes. Presentation to most clones was detectable after 60 minutes of antigen pulsing in the absence of competing peptide but in the presence of AENK, presentation to some clones was either undetectable (AK 20 and AK 90; Fig 3b) or profoundly inhibited (eg AK 6). At later times there was some recovery of presentation of these epitopes as expected for a competitive inhibitor. Several controls ruled out the possibility that the inhibitory effect of the AENK competitor is simply due to non-specific toxicity. First, presentation to other clones recognising other regions of TTCF was not affected by the AENK competitor (eg AK 111, 71 and 33: Fig 3c). Secondly, there was a clear differential sensitivity to AENK versus AEQK (Fig 3c), strongly suggesting that specific inhibition of AEP was responsible for the slowed kinetics of presentation. Thirdly, the inhibitory effects of AENK could be overridden by using TTCF pre-digested *in vitro* by AEP. In other words, the requirement for AEP *in vivo,* and hence the inhibitory effect of AENK, was by-passed by AEP cleavage *in vitro* (data not shown). Taken together our results reveal AEP as a new and highly specific processing activity in the class II MHC pathway. Preliminary studies show that AEP is present in a variety of other antigen presenting cell types.

Although we uncovered this processing activity using an antigen proteolysis assay, it is possible that it also plays a role in invariant chain processing as well. Splenocytes from cathepsin D gene targeted mice continued to produce invariant chain processing products even when all known cysteine protease activity were also suppressed by leupeptin⁷. Villadangos *et al* suggested that other non-cysteine proteases (besides cathepsin D) must be initiating Ii (invariant chain) cleavage. Cathepsin E is one possible candidate but so too is AEP since, although it is a cysteine protease, it is completely resistant to leupeptin (Fig 1a). Intact p31 Ii is readily cleaved by AEP *in vitro* and we are currently assessing the possibility that AENK also interferes with Ii processing. However, the differential effect of the AENK inhibitor on T cell clones and the fact that pre-digestion of antigen with AEP abolished the inhibitory effect of the AENK inhibitor argues that its primary inhibitory effect in our studies was on TTCF processing.

Can any special significance be attached to the existence of an asparaginyl endopeptidase in antigen presenting cells? One possibility is that microbial proteins might in general be better substrates than host proteins since the latter could resist AEP attack by N-glycosylation of particularly sensitive asparagine residues. It is very striking that while TTCF is an extremely good substrate for AEP, cleavage occurs at only 3 of its 47 asparagine residues. This suggests that there may be additional determinants of AEP specificity to be identified and that strategic N-glycosylation might give substantial protection. Thus the innate immune systems ability to bind microbial antigen through recognition of non-self molecular 'patterns', might be harnessed to an 'innate' bias towards microbial antigen processing as well. In addition, our data raise the possibility that disturbances of N-glycosylation might result in altered processing of self proteins by AEP leading to presentation of 'cryptic' epitopes with possible pathological consequences.

### Methods

*TTCF processing activity.* Lysosomal fractions from the human B cell line EDR were prepared on 27 % Percoll density gradients as previously described⁸ using ¹²⁵I-labelled transferrin (internalised for 30 minutes at 37°C) and β-hexosaminidase to identify endosomal/plasma membrane and lysosomal fractions respectively. The peak of lysosomes was collected by centrifugation at 40,000 g for 1 hour, removed from the underlying Percoll pellet, collected again at 50,000 g for 1 hour and stored frozen. For separation of leupeptin-insensitive cysteine protease activity, 7.5 x 10⁹ cells were homogenised in a ball-bearing homogenizer and nuclei and unbroken cells removed by centrifugation at 2,000 g for 10 minutes. A membrane pellet was collected at 40,000 g and solubilised in 50 mM citrate buffer, pH 5.5 containing 0.1% CHAPS (3-([cholamidopropyl)dimethylammonio]-1-propane-sulphonate). The extract was centrifuged at 2,000 g for 20 minutes and then applied to a Mono-S column (Pharmacia). Fractions were eluted with a gradient of NaCI and monitored for TTCF processing activity.

*Proteins and peptides.* A histidine tagged derivative of the C terminal domain of tetanus toxin was prepared^{9,10} and purified¹⁰ as described. This protein has residues 872-1315 of the complete toxin (1-1315) preceded by the sequence MGHGHHHHHHHHHHSSGHIEGRHI. Mutagenesis of residues 1184/5 was performed using template EH106 obtained by cloning the His-TTCF (Reference 10) as an XbaI/BamHI fragment into pSL1180 (Pharmacia). Site directed mutagenesis of residues 1184/85 was performed according to the method of Mikaelian and Sergent¹¹ using the mutagenic primer CGC TAC ACT CCG AAC GCG GCG ATC GAT TCT TTC GTT and flanking primers M13 rev (AGCGGATAACAATTTCACACAGGA) and M13 seq (GTAAAACGACGGCCAGT). After sequencing to confirm mutagenesis, recloned back into pET16B for expression. Tetrapeptides were synthesised using Fmoc chemistry leaving the C-terminus amidated and the N-terminus retaining the Fmoc group. LHVS was a kind gift from Hidde Ploegh, MIT.

*Antigen presentation.* Peripheral blood mononuclear cells were prepared by Ficoll/Paque centrifugation and used fresh. T cell clones specific for TTCF were established from donor A.K. according to¹². Epitope mapping was performed using a set of 88 peptides, 17 residues in length spanning the TTCF sequence (Chiron Mimotopes). Clones AK 6, 90, and 71 recognise the peptides 1235-1245, 1145-1156 and 950-961 respectively. Clones 111 and 33 recognise epitopes within the regions 1225-1276 and 1104-1153. The epitope recognised by clone 20 has not been mapped. Peptides AENK or AEQK were dissolved in water, made isotonic with NaCl and diluted into RPMI growth medium. T cell proliferation assays were performed essentially as described^{12,13}. Briefly, following antigen pulsing (30 µg/ml TTCF) with or without tetrapeptides (1mg/ml) PBMC were washed in PBS and fixed for 45 seconds in 0.05 % glutaraldehyde. Glycine was added to a final concentration of 0.1M and the cells washed 5 times in RPMI 1640 medium containing 1 % FCS before co-culture with T cell clones in round-bottom 96-well microtitre plates. After 48 hours the cultures were pulsed with 1 µCi of ³H-thymidine and harvested 16 hours later.

Further experiments showed that AEP plays a role in TTCF processing *in vivo.* Freshly isolated peripheral blood mononuclear cells (PBMC) were pre-incubated briefly with AENK, or the control peptide AEQK, and then pulsed with TTCF antigen in the continued presence of one or other peptide. At different times, the cells were washed, fixed and then co-cultured with different autologous T cell clones to assess expression of different peptide/MHC complexes. Presentation was detectable between 30 and 60 minutes of antigen pulsing in the presence of the control peptide (Fig 9a) but in the presence of AENK, presentation to most clones (eg AK 5, 90 & 6) was undetactable at these early times. We also generated an EBV-B cell line from donor AK and tested the effect of the tetrapeptides on B cell antigen presentation to the same T cell clones. As shown in Figure 9b, the AENK peptide blocked antigen presentation by EBV-B cells very effectively. The control peptide AEQK, had virtually no effect on the kinetics of antigen presentation (Fig 9b). Thus AENK specifically inhibited antigen presentation by two different APC populations. Antigen presentation to all clones recovered to different extents after longer times of antigen pulsing. We suggest that this recovery is expected given that AENK inhibits by acting as a competitive substrate and is itself hydrolysed by asparaginyl endopeptidases (data not shown). Under these conditions it is not possible to block TTCF processing completely. We also observed that some clones, for example clone 33 were less sensitive to blockade of AEP (Fig 4a & b). Clonal variations such as these might be explained either by a differential requirement for AEP processing, dependent on the location of the T cell epitope or on the antigen dose/response of individual clones or on a combination of these factors. Consistent with the first scenario, we have observed that clones specific for the same peptide region do show similar sensitivites to AEP blockade. For example, clones 5 & 90 and a third clone (AK 20) all recognise the 1145-1156 peptide and show very similar sensitivity to AENK (Fig 9 and data not shown). However, as expected, there was also variation in the antigen dose/response among the clones we tested. Those requiring higher levels of antigen for half-maximal triggering generally showed slower kinetics of presentation and greater sensitivity to AEP blockade. We suspect that the apparent differential requirement for AEP processing shown by different clones is due to a combination of epitope location, clone 'affinity' and possibly other factors. Nonetheless, it is clear that presentation to most tetanus toxin cell epitopes tested to date is affected by AEP blockade. This is consistent with the idea that initial processing by AEP may be the 'key' which 'unlocks' the native protein for further processing. As a further test of this hypothesis, we assayed the kinetics of presentation of TTCF that had been pre-digested *in vitro* with AEP, reasoning that this might accelerate antigen presentation. As shown in Figure 9c, this was indeed the case. Cells incubated with TTCF pre-processed with AEP presented T cell epitopes after only 15-30 minutes whereas unprocessed TTCF required 60-120 minutes before presentation could be detected. Importantly, AENK did not block the rapid presentation of the pre-processed antigen at these times (Fig 9d), confirming that this inhibitor specifically blocks AEP action on the antigen substrate *in vivo.* TTCF pre-processed with AEP still required further cellular processing since it could not be presented by fixed PBMC (Fig 9c & d). Taken together these results support a working model whereby AEP initiates TTCF proteolysis but that generation of suitable peptides for MHC binding requires further intracellular processing. Native TTCF was a poor substrate for cathepsins E,D,L,S and B (data not shown) but many processing sites, for example for cathepsins E and D were revealed following antigen unfolding.

We also analysed a natural 21-residue tryptic glycopeptide (residues 622-642) purified from human serum transferrin (see Table 1). This peptide contains 2 asparagine residues one of which (N630) carries a biantennary oligosaccharide (Lu, J. & van Halbeek, H. (1996) "Complete 1H and 13C resonance assignments of a 21-amino acid glycopeptide prepared from human serum transferrin." Carbohydr. Res. 296, 1-21). The same peptide was synthesised without modification of the asparagines and digested in parallel. [3 lysine residues were added to improve solubility].

Both mass spectrometric analysis and N-terminal sequencing of the products confirmed that hydrolysis of both asparagines 630 and 637 occurred in the non-glycosylated peptide (Table 1). However, we only detected products from hydrolysis of N637 in the glycopeptide (Table 1). A second transferrin-derived tryptic glycopeptide (residues 421-452) containing 3 asparagine residues was also digested with AEP. Again, we only detected fragments from hydrolysis at the non-glycosylated asparagines 430 & 436 (not shown).

**Table 1: N-glycosylation blocks asparaginyl endopeptidase action on a natural glycopeptide**

| | | **Predicted Mass** | **Observed Mass** |
|---|---|---|---|
| Synthetic Transferrin Peptide (622-642KKK) | | | |
| No enzyme | | | |
| | QQQHLFGSNVTDCSGNFCLFRKKK | 2784.36 | 2784.17 |
| + Asparagine endopeptidase | | | |
| | QQQHLFGSN | 1058.51 | 1058.46 |
| | VTDCSGNFCLFRKKK | 1745.83 | 1743.69* |
| | FCLFRKKK | 1069.63 | 1069.59 |
| Transferrin glycopeptide (622-642) | | | |
| No Enzyme | | | |
| | QQQHLFGSN(CHO)VTDCSGNFCLFR | 4720.76 | 4720.2 |
| | | | |
| + Asparagine endopeptidase | | | |
| | | | |
| | QQQHLFGSN(CHO)VTDCSGN | 3996.88 | 3996.85 |
| | FCLFR | 742.91 | 742.23 |
| | VTDCSGNFCLFR | 1476.66 | not observed |
| | QQQHLFGSN(CHO) | 3263.13 | not observed |

Predicted fragment masses were obtained from the human transferrin sequence (accession number: P02787) calculated using PeptideMass at the EXPASY server. The transferrin biantennary oligosaccharide (2 Sialic acid + 4 GIcNAc + 5 hexose has a mass of 2204 coupled to peptide Lu, J. & van Halbeek, H. (1996) "Complete 1H and 13C resonance assignments of a 21-amino acid glycopeptide prepared from human serum transferrin." Carbohydr. Res. 296, 1-21). Variants also observed but not shown are monosialylated forms of the glycopeptides and N-terminal peptide forms 17 mass units lower corresponding to N-terminal glutamine pyroglutamisation. *Cyclisation of the two cysteine residues in this peptide likely accounts for the observed mass deficit of 2.

### Example 2: Asparaginyl endopeptidase can process the Class II MHC invariant chain

A key step in the generation of peptide/class II MHC complexes is the complete removal of a protein called the invariant chain from the class II MHC αβ dimer. This is a dedicated chaperone for class II MHC which must be removed by proteolysis before any peptide can be loaded (Cresswell (1996) Cell 84, 505-). Normally, its removal requires, first, proteolysis to produce a small fragment (known as CLIP ^{∼}about 3 kD) which still blocks the class II peptide binding site, and second, the action of a protein called DM which removes this peptide. Blocking invariant chain removal effectively shuts down loading of all other peptides. There are two alternatively spliced forms of the invariant chain usually known as p31 and p41. p41 is minor in most cells except dendritic cells where it accounts for around 40 % of invariant chain.

*In vitro,* cathepsin S *can* produce the small fragment (CLIP) suitable for DM action (Riese et al (1996) Immunity 4, 357-) but it is likely that other enzymes are involved in invariant chain processing in living cells. For example, inhibitors of cathepsin S result in the accumulation of an invariant chain fragment considerably larger (^{∼} 10 kD) than CLIP. This shows that cathepsin S is only really essential at the later stages of invariant chain processing and that other enzymes can produce the 10 kD fragment. It has now been found that AEP can degrade the invariant chain *in vitro.* Thus, AEP processing of the invariant chain may initiate its destruction in some or all cells (some cells, eg thymic cortical epithelial cells do not seem to express cathepsin S).

The human EBV-transformed lines PALA or AL-EBV were radiolabelled for 15 minutes at 37°C with 0.5 mCi/ml ³⁵S-Translabel (ICN), washed and lysed in Tris-buffered saline containing 1% Triton X-100 plus protease inhibitors (Davidson & Watts (1989) J. Cell. Biol. 109, 85). The lysates were cleared by centrifugation and pre-cleared with Protein G sepharose. Invariant chain/class II MHC complexes were precipitated with the VIC-Y1 antibody (gift from W Knapp). The complexes were collected on Protein G sepharose, washed 3 times in Tris-buffered saline, 1 % Triton X-100 and resuspended in 50 mM citrate buffer, 1 mM EDTA, 5 mM dithiothreitol with or without 0.05 % CHAPS (Calbiochem). Purified aspariginyl endopeptidase was added to 5 mU per 20 µl reaction. Digestions were performed at 37°C for 30 minutes and analysed by Tris-tricine SDS-PAGE (12%). Note that both p41 and p31 forms of the invariant chain are completely digested to produce 3-4 discrete fragments (Figure 6) while both the α and β chains of class II MHC are resistant to AEP processing. The presence of the detergent CHAPS does not affect the digestion.

Circumstantial evidence has previously shown that enzymes, other than cathepsin S, L, B and D, can degrade the invariant chain in living cells. This comes from the finding that in spleen cells from a cathepsin D knock-out mouse also treated with leupeptin (which inhibits cathepsins S, L and B), the early stages of invariant chain processing still proceed (Villadangos et al (1997) J. Exp. Med. 186, 549-). The results presented here suggest that AEP could be responsible for this residual invariant chain processing activity.

A further experiment was carried out to demonstrate invariant chain processing. The results are shown in Figure 7.

The human EBV-transformed cell line Pala was labelled with ³⁵S-Methionine/Cysteine (Translabel, ICN) for 30 minutes and the cells lysed for immunoprecipitation. Cleared lysates were incubated with either mAb DA6.231 (class II MHC β-chain specific) or VIC-γ1 (invariant chain specific). Recovered immunoprecipitates were then digested with 5 mU purified pig kidney legumain for 60 minutes. The digests were then run on 12 % Tris-Tricine SDS gels, transferred to PVDF membrane and the numbered fragments in panel (a) of Figure 7 arising from AEP processing subjected to N-terminal Edman degradation. The eluate from each cycle of cleavage was dried down, resuspended in scintillation cocktail and counted on a β counter. Similar digestion patterns were obtained for the DA6.231 precipitates (not shown). ³⁵S cpm released at specific cycles of Edman degradation allowed the Asparagine residues cleaved to be identified. An illustrative example of the raw data obtained from sequencing band 5 is shown in (b) of Figure 7. Figure 7(c) Human li p31 sequence showing 3 AEP processing sites. AEP can cleave on both sides of the CLIP region which is underlined. Note that the first site obtained from sequencing band 1 is in the cytoplasmic tail and would not be available for processing in living cells.

### References for Example 1

1. Chen, J.M., et al (1997) J. Biol. Chem. 272, 8090-8.
2. Kembhavi, A.A., Buttle, D.J., Knight, C.G. & Barrett, A.J. (1993) Arch. Biochem. Biophys. 303, 208-13.
3. Dalton, J.P., Hola Jamriska, L. & Brindley, P.J. (1995) Parasitology 111, 575-80.
4. Riese, R.J., et al (1996) Immunity 4, 357-66.
5. Watts, C. (1997) Annu Rev Immunol 15, 821-50.
6. Fineschi, B. & Miller, J. (1997) Trends Biochem Sci 22, 377-382.
7. Villadangos, J.A., Riese, R.J., Peters, C., Chapman, H.A. & Ploegh, H.L. (1997) J. Exp. Med. 186, 549-60.
8. Davidson, H.W., West, M.A. & Watts, C. (1990) J Immunol 144, 4101-9.
9. Makoff, A.J., Ballantine, S.P., Smallwood, A.E. & Fairweather, N.F. (1989) BiolTechnology 7, 1043-1046.
10. Hewitt, E.W., et al (1997) J. Immunol. 159, 4693-9.
11. Mikelain, I. & Sergent, A. (1992) Nucleic Acids Res. 20, 376.
12. Lanzavecchia, A. (1985) Nature 314, 537-539.
13. Pond, L. & Watts, C. (1997) J. Immunol. 159, 543-53.

### References for synthetic methods desribed in Figure 4

1. Thomson, R. C. Meth. Enzymol. 46, 220-
2. Vinitsky, A., Cardozo, C., Sepp-Lorenzino, L, Michaud, C. & Orlowski, M. Inhibition of the proteolytic activity of the multicatalytic proteinase complex by Substrate-related peptidyl aldehydes. J. Biol. Chem. 269, 29860-29866 (1994).
3. Shaw, E. & Green, G.D.J. Inactivation of thiol proteases with peptidyl diazomethyl ketones. Meth. Enzymol. 80, 820-826 (1981).
4. Shaw, E. Peptidyl diazomethanes as inhibitors of cysteine and serine proteases. Meth. Enzymol. 244, 649-656 (1994).
5. Williams, E.B. & Mann, K.G. Peptide chloromethyl ketones as labelling reagents. Meth. Enzymol. 222, 503-513 (1993).
6. Palmer, J.T., Rasnick, D., Klaus, J.L. & Bromme, D. Vinyl sulphones as mechanism-based cystein protease inhibitors. J. Med. Chem. 38, 3193-96 (1995).
7. Krantz, A. Peptidyl (acyloxy) methanes as quiescent affinity labels for cysteine proteases. Meth. Enzymol. 244, 656-671 (1994).
8. Bromme, D. & Memuth, H-U. N, O-diacyl hydroxamates as selective and irreversible inhibitors of cystein proteases. Meth. Enzymol. 244, 671-685.

## Claims

1. An *in vitro* method of reducing the processing of a protein antigen, or reducing presentation of a protein antigen by a MHC Class II molecule, by a cell, the method comprising contacting the cell with a selective inhibitor of asparaginyl endopeptidase wherein the selective inhibitor;
(i) is an N and C-terminal blocked peptide Ala-Glu-Asn-Lys-NH (AENK) or Lys-Asn-Asn-Glu-NH (KNNE); or
(ii) has the structure Bl-(X)ₙ-Asn-Q where Bl is any suitable N terminal blocking group; X is an amino acid residue; n is between 1 and 100, Asn is an asparagine residue and Q is selected from the groups consisting of aldehydes; halomethyl ketones; chloromethylketone; fluoromethylketone; diazomethane (diazomethyl ketone); vinyl sulphone-R (wherein R is any suitable alkyl or aryl terminating group and includes C₁ to C₁₀ alkyl, phenyl, benzyl, naphthyl and the like); (acyloxy)methanes; N, O diacyl hydroxamates; nitriles; α-keto carbonyls; ketomethylsulfonium salts; or epoxides.

2. Use of a selective inhibitor of asparaginyl endopeptidase in the manufacture of a medicament for use as an immunosuppressive agent, wherein the selective inhibitor;
(i) is an N and C-terminal blocked peptide Ala-Glu-Asn-Lys-NH (AENK) or Lys-Asn-Asn-Glu-NH (KNNE); or
(ii) has the structure B1-(X)ₙ-Asn-Q where Bl is any suitable N terminal blocking group; X is an amino acid residue; n is between 1 and 100, Asn is an asparagine residue and Q is selected from the groups consisting of aldehydes; halomethyl ketones; chloromethylketone; fluoromethylketone; diazomethane (diazomethyl ketone); vinyl sulphone-R (wherein R is any suitable alkyl or aryl terminating group and includes C₁ to C₁₀ alkyl, phenyl, benzyl, naphthyl and the like); (acyloxy)methanes; N, O diacyl hydroxamates; nitriles; α-keto carbonyls; ketomethylsulfonium salts; or epoxides.

3. The use according to claim 2 wherein the medicament is for the treatment of an autoimmune, allergic, hypersensitivity or transplant rejection disorder.

4. A method according to Claim 1 wherein the cell is, or is comprised in a tissue or organ, for transplantation into a patient.

5. A method of identifying a compound for modulating Class II MHC antigen processing the method comprising contacting a test compound with asparaginyl endopeptidase and selecting a compound which reduces its activity, and determining whether the so selected compound is capable of substantially inhibiting the loading and presentation of peptides on an appropriate Class II MHC molecule-containing cell.

6. A method according to Claim 5 wherein the activity of asparaginyl endopeptidase is measured using a substrate which upon cleavage by said endopeptidase, yields a readily detectable product.

7. A method according to Claim 6 wherein the substrate is Z-Ala-Ala-Asn-7-(4-methyl)coumarylamide and the product is fluorescent.

8. A method according to Claim 6 wherein it is determined whether the so selected compound is capable of substantially inhibiting T cell activation by an appropriate Class II MHC molecule-containing cell.

9. A non-human transgenic animal wherein a gene encoding asparaginyl endopeptidase has been modified and the animal expresses substantially no asparaginyl endopeptidase from said gene.

10. A non-human transgenic animal according to Claim 9 which is a mouse.

11. A non-human transgenic animal according to Claim 9 or 10 further comprising a genetic background which predisposes to an autoimmune disease either spontaneously or upon administration of protein antigen.

12. A non-human transgenic animal according to Claims 9 or 10 further transgenic for a human Class II MHC molecule and, optionally, further transgenic for human CD4.

13. An inhibitor of asparaginyl endopeptidase which has the structure B1-(XₐXₙ)-Asn-Q wherein Bl is any suitable N terminal blocking group; XₐXₙare the n amino acid residues immediately N terminal to an Asn cleavage site in the invariant chain of Class II MHC molecules; Asn is an asparagine residue; and Q is selected from the groups consisting of aldehydes; halomethyl ketones; chloromethylketone; fluoromethylketone; diazomethane (diazomethyl ketone); vinyl sulphone-R (wherein R is any suitable alkyl or aryl terminating group and includes C₁ to C₁₀ alkyl, phenyl, benzyl, naphthyl and the like); (acyloxy)methanes; N, O diacyl hydroxamates; nitriles; α-keto carbonyls; ketomethylsulfonium salts; or epoxides; and n is an integer from 1 to 100.

14. An inhibitor according to Claim 13 wherein the number of amino acid residues in (XₐXₙ is between 1 and 25, preferably between 2 and 10.

15. An inhibitor according to Claim 14 which is any of B1-Ser-Gln-Asn-Q; Bl-Leu-Glu-Asn-Q; Bl-Leu-Gln-Asn-Q; Bl-Pro-Glu-Asn-Q; B1-Leu Lys-Asn-Q; B1-Gln-Asn-Q; B1-Glu-Asn-Q; B1-Asp-Glu-Asn-Q; Bl-Asn-Gly-Asn-Q; Bl-Phe-Pro-Asn-Q; B1-Val-Pro-Asn-Q: and B1-His-His-Asn-Q.

16. An inhibitor of asparaginyl endopeptidase which has the structure (Xb-Xc)Asn(Xd-Xe) wherein (Xb-Xc) are the r amino acid residues immediately N terminal to an Asn cleavage site in the invariant chain of Class II MHC molecules and (Xd-Xe) are the s amino acid residues immediately C terminal to an Asn cleavage site in the said invariant chain; Asn is an asparagine residue; and r and s are independently between 2 and 25, provided that the inhibitor is not the peptide CVFPNGTEVPNTRSRGHHN or the peptide ATKYGNMTEDHVMHLLQNA.

17. An inhibitor which has the structure (Xb-Xc)Asn(Xd-Xe) wherein (Xb-Xc) are the r amino acid residues immediately N terminal to an Asn cleavage site in the invariant chain of Class II MHC molecules and (Xd-Xe) are the s amino acid residues immediately C terminal to an Asn cleavage site in the said invariant chain; Asn is an asparagine residue; and r and s are independently between 2 and 5.

18. An inhibitor according to any one of Claims 13 to 15 or an inhibitor which has the structure (Xb-Xc)Asn(Xd-Xe) wherein (Xb-Xc) are the r amino acid residues immediately N terminal to an Asn cleavage site in the invariant chain of Class II MHC molecules and (Xd-Xe) are the s amino acid residues immediately C terminal to an Asn cleavage site in the said invariant chain; Asn is an asparagine residue; and r and s are independently between 2 and 25, for use in medicine.

19. A method according to Claim 1 or a use according to Claims 2 or 3 wherein the inhibitor is an inhibitor according to any one of Claims 13 to 18.

## Patentansprüche

1. *In vitro* durchzuführendes Verfahren zur Verminderung der Verarbeitung eines Proteinantigens oder zur Verminderung der Präsentation eines Proteinantigens durch ein Klasse-II-MHC-Molekül durch eine Zelle, wobei das Verfahren das in Berührung bringen der Zelle mit einem selektiven Inhibitor von Asparaginylendopeptidase umfasst, wobei der selektive Inhibitor
(i) ein N- und C-terminal blockiertes Peptid Ala-Glu-Asn-Lys-NH (AENK) oder Lys-Asn-Asn-Glu-NH (KNNE) ist; oder
(ii) die Struktur Bi-(X),-Asn-Q aufweist, wobei BI irgendeine geeignete N-terminal blockierende Gruppe und X ein Aminosäure-Residuum ist, n zwischen 1 und 100 liegt, Asn ein Asparagin-Residuum ist und Q aus der Gruppe ausgewählt ist, die aus Aldehyden, Halomethylketonen, Chlormethylketon, Fluormethylketon, Diazomethan (Diazomethylketon), Vinylsulfon-R (wobei R irgendeine geeignete Alkyl- oder Aryl-Abschlussgruppe ist und C₁- bis C₁₀-Alkyl-, -Phenyl-, -Benzyl-, -Naphthyl-Gruppen und dergleichen aufweist), Alkoxymethane, N-, O-Diazylhydroxamate, Nitrile, α-Keto-Carbonyle, Ketomethylsulfoniumsalze oder -Epoxide umfasst.

2. Verwendung eines selektiven Inhibitors von Asparaginylendopeptidase bei der Herstellung eines Medikamentes zur Verwendung als immunosuppressiver Wirkstoff, wobei der selektive Inhibitor
(i) ein N- und C-terminal blockiertes Peptid Ala-Glu-Asn-Lys-NH (AENK) oder Lys-Asn-Asn-Glu-NH (KNNE) ist; oder
(ii) die Struktur Bl-(X)ₙ-Asn-Q aufweist, wobei Bl irgendeine geeignete N-terminal blockierende Gruppe und X ein Aminosäure-Residuum ist, n zwischen 1 und 100 liegt, Asn ein Asparagin-Residuum ist und Q aus der Gruppe ausgewählt ist, die aus Aldehyden, Halomethylketonen, Chlormethylketon, Fluormethylketon, Diazomethan (Diazomethylketon), Vinylsulfon-R (wobei R irgendeine geeignete Alkyl- oder Aryl-Abschlussgruppe ist und C₁- bis C₁₀-Alkyl-, -Phenyl-, -Benzyl-, -Naphthyl-Gruppen und dergleichen aufweist), Alkoxymethane, N-, O-Diazylhydroxamate, Nitrile, α-Keto-Carbonyle, Ketomethylsulfoniumsalze oder -Epoxide umfasst.

3. Verwendung nach Anspruch 2, wobei das Medikament zur Behandlung einer Autoimmun-Störung, einer allergischen Störung, von Hypersensitivität oder einer Transplantat-Abstoßung dient.

4. Verfahren nach Anspruch 1, wobei die Zelle für eine Transplantation in einen Patienten dient oder Bestandteil eines Gewebes oder eines Organs für eine Transplantation in einem Patienten ist.

5. Verfahren zur Identifizierung einer Verbindung zur Veränderung der Verarbeitung des Klasse-II-MHC-Antigens, wobei das Verfahren das in Berührung bringen einer Testverbindung mit Asparaginylendopeptidase und die Auswahl einer Verbindung umfasst, die dessen Aktivität vermindert, sowie die Ermittlung, ob die so ausgewählte Verbindung in der Lage ist, im wesentlichen die Beladung und Präsentation von Peptiden an einer geeigneten, ein Klasse-II-MHC-Molekül enthaltenden Zelle zu hemmen.

6. Verfahren nach Anspruch 5, bei dem die Aktivität von Asparaginylendopeptidase unter Verwendung eines Substrats gemessen wird, das bei einer Spaltung durch diese Endopeptidase ein ohne weiteres erkennbares Produkt liefert.

7. Verfahren nach Anspruch 6, bei dem das Substrat Z-Ala-Ala-Asn-7-(4-methyl)coumarylamid und das Produkt fluoreszierend ist.

8. Verfahren nach Anspruch 6, bei dem ermittelt wird, ob die so ausgewählte Verbindung in der Lage ist, im wesentlichen eine T-Zellen-Aktivierung durch eine geeignete ein Klasse-II-MHC-Molekül enthaltende Zelle zu hemmen.

9. Nicht menschliches transgenes Tier, bei dem ein Asparaginylendopeptidase kodierendes Gen modifiziert worden ist und das Tier im Wesentlichen aus diesem Gen keine Asparaginylendopeptidase exprimiert.

10. Nicht menschliches transgenes Tier nach Anspruch 9, bei dem es sich um eine Maus handelt.

11. Nicht menschliches transgenes Tier nach Anspruch 9 oder 10, das weiterhin einen genetischen Hintergrund aufweist, der eine Prädisposition für eine Autoimmun-Störung entweder spontan oder bei der Verabreichung eines Proteinantigens beinhaltet.

12. Nicht menschliches transgenes Tier nach Anspruch 9 oder 10, das weiterhin für ein menschliches Klasse-II-MHC-Molekül und optional weiterhin transgen für menschliches CD4 ist.

13. Inhibitor von Asparaginylendopeptidase, der die Struktur Bl-(Xₐ-Xₙ)-Asn-Q aufweist, wobei BI irgendeine geeignete N-terminal blockierende Gruppe ist, XₐXₙ die n-Aminosäure-Residuen sind, die unmittelbar N-terminal zu einer Asn-Spaltungsstelle in der invarianten Kette von Klasse-II-MHC-Molekülen sind, Asn ein Asparagin-Residuum ist und Q aus der Gruppe ausgewählt ist, die aus Aldehyden, Halomethylketonen, Chlormethylketon, Fluormethylketon, Diazomethan (Diazomethylketon), Vinylsulfon-R (wobei R irgendeine geeignete Alkyl- oder Aryl-Abschlussgruppe ist und C₁- bis C₁₀-Alkyl-, -Phenyl-, -Benzyl-, -Naphthyl-Gruppen und dergleichen aufweist), Alkoxymethane, N-, O-Diazylhydroxamate, Nitrile, α-Keto-Carbonyle, Ketomethylsulfoniumsalze oder -Epoxide umfasst, und wobei n eine ganz Zahl von 1 bis 100 ist.

14. Inhibitor nach Anspruch 13, bei dem die Anzahl von Aminosäure-Residuen in (XₐXₙ) zwischen 1 und 25 und vorzugsweise zwischen 2 und 10 liegt.

15. Inhibitor nach Anspruch 14, bei dem es sich um eine der folgenden Substanzen handelt: BI-Ser-Gln-Asn-Q, Bl-Leu-Glu-Asn-Q, BI-Leu-Gln-Asn-Q, BI-Pro-Glu-Asn-Q, Bl-Leu-Lys-Asn-Q, BI-Gln-Asn-Q, BI-Glu-Asn-Q, Bl-Asp-Glu-Asn-Q, BI-Asn-Gly-Asn-Q, BI-Phe-Pro-Asn-Q, Bl-Val-Pro-Asn-Q, und BI-His-His-Asn-Q.

16. Inhibitor von Asparaginylendopeptidase, der die Struktur (Xb-Xc)Asn(Xd-Xe) aufweist, wobei (Xb-Xc) die r-Aminosäure-Residuen unmittelbar N-terminal zu einer Asn-Spaltungsstelle in der invarianten Kette von Klasse-II-MHC-Molekülen und (Xd-Xe) die s-Aminosäure-Residuen unmittelbar C-terminal zu einer Asn-Spaltungsstelle in dieser invarianten Kette sind, Asn ein Asparagin-Residuum ist und r und s unabhängig voneinander zwischen 2 und 25 liegen, vorausgesetzt, dass der Inhibitor nicht das Peptid CVFPNGTEVPNTRSRGHHN oder das Peptid ATKYGNMTEDHVMHLLQNA ist.

17. Inhibitor, der die Struktur (Xb-Xc)Asn(Xd-Xe) aufweist, wobei (Xb-Xc) die r-Aminosäure-Residuen unmittelbar N-terminal zu einer Asn-Spaltungsstelle in der invarianten Kette von Klasse-II-MHC-Molekülen und (Xd-Xe) die s-Aminosäure-Residuen unmittelbar C-terminal zu einer Asn-Spaltungsstelle in dieser invarianten Kette sind, Asn ein Asparagin-Residuum ist und r und s unabhängig voneinander zwischen 2 und 5 liegen.

18. Inhibitor nach einem der Ansprüche 13 bis 15 oder Inhibitor, der die Struktur (Xb-Xc)Asn(Xd-Xe) besitzt, wobei (Xb-Xc) die r-Aminosäure-Residuen unmittelbar N-terminal zu einer Asn-Spaltungsstelle in der invarianten Kette von Klasse-II-MHC-Molekülen und (Xd-Xe) die s-Aminosäure-Residuen unmittelbar C-terminal zu einer Asn-Spaltungsstelle in dieser invarianten Kette sind, Asn ein Asparagin-Residuum ist und r und s unabhängig voneinander zwischen 2 und 25 liegen zur Verwendung in der Medizin.

19. Verfahren nach Anspruch 1 oder Verwendung nach Anspruch 2 oder 3, wobei der Inhibitor ein Inhibitor nach einem der Ansprüche 13 bis 18 ist.

## Revendications

1. Procédé *in vitro* pour réduire le traitement d'un antigène protéique, ou réduire la présentation d'un antigène protéique par une molécule du CMH de classe II, par une cellule, le procédé comprenant la mise en contact de la cellule avec un inhibiteur sélectif de l'asparaginyl endopeptidase, dans lequel l'inhibiteur sélectif ;
(i) est un peptide à blocage N- ou C-terminal Ala-Glu-Asn-Lys-NH (AENK) ou Lys-Asn-Asn-Glu-NH (KNNE) ; ou
(ii) a la structure B1-(X)ₙ-Asn-Q où B1 est tout groupe bloquant N-terminal approprié ; X est un résidu d'acide aminé ; n est entre 1 et 100, Asn est un résidu asparagine et Q est choisi dans les groupes constitués par les aldéhydes ; les halogénométhyl cétones; la chlorométhylcétone; la fluorométhylcétone; le diazométhane (diazométhyl cétone) ; la vinyl sulfone-R (où R est tout groupe terminal alkyle ou aryle approprié et inclut alkyle en C₁ à C₁₀, phényle, benzyle, naphtyle et similaires) ; les (acyloxy)-méthanes ; les N, O diacyl hydroxamates; les nitriles ; les α-céto carbonyles ; les sels de céto-méthylsulfonium ; ou les époxydes.

2. Utilisation d'un inhibiteur sélectif de l'asparaginyl endopeptidase dans la fabrication d'un médicament pour une utilisation en tant qu'agent immunosuppresseur, dans laquelle l'inhibiteur sélectif ;
(i) est un peptide à blocage N- ou C-terminal Ala-Glu-Asn-Lys-NH (AENK) ou Lys-Asn-Asn-Glu-NH (KNNE) ; ou
(ii) a la structure B1-(X)ₙ-Asn-Q où B1 est tout groupe bloquant N-terminal approprié ; X est un résidu d'acide aminé ; n est entre 1 et 100, Asn est un résidu asparagine et Q est choisi dans les groupes constitués par les aldéhydes ; les halogénométhyl cétones ; la chlorométhylcétone; la fluorométhylcétone ; le diazométhane (diazométhyl cétone) ; la vinyl sulfone-R (où R est tout groupe terminal alkyle ou aryle approprié et inclut alkyle en C₁ à C₁₀, phényle, benzyle, naphtyle et similaires); les (acyloxy)-méthanes ; les N, O diacyl hydroxamates; les nitriles ; les α-céto carbonyles ; les sels de céto-méthylsulfonium ; ou les époxydes.

3. Utilisation selon la revendication 2, dans laquelle le médicament est destiné au traitement d'un trouble auto-immun, allergique, d'hypersensibilité ou de rejet de greffon.

4. Procédé selon la revendication 1, dans lequel la cellule est, ou est comprise dans, un tissu ou un organe, pour la transplantation dans un patient.

5. Procédé d'identification d'un composé pour moduler le traitement d'un antigène du CMH de classe II, le procédé comprenant la mise en contact d'un composé d'essai avec de l'asparaginyl endopeptidase et le choix d'un composé qui réduit son activité, et le fait de déterminer si le composé ainsi choisi est apte à inhiber substantiellement la charge et la présentation de peptides sur une cellule contenant une molécule du CMH de classe II appropriée.

6. Procédé selon la revendication 5, dans lequel l'activité de l'asparaginyl endopeptidase est mesurée en utilisant un substrat qui, par clivage par ladite endopeptidase, donne un produit aisément détectable.

7. Procédé selon la revendication 6, dans lequel le substrat est le Z-Ala-Ala-Asn-7-(4-méthyl)coumaryl-amide et le produit est fluorescent.

8. Procédé selon la revendication 6, dans lequel il est déterminé si le composé ainsi choisi est apte à inhiber substantiellement l'activation des lymphocytes T par une cellule contenant une molécule du CMH de classe II appropriée.

9. Animal transgénique non humain, dans lequel un gène codant l'asparaginyl endopeptidase a été modifié et l'animal n'exprime pratiquement pas d'asparaginyl endopeptidase à partir dudit gène.

10. Animal transgénique non humain selon la revendication 9, qui est une souris.

11. Animal transgénique non humain selon la revendication 9 ou 10, comprenant en outre un contexte génétique qui prédispose à une maladie auto-immune soit spontanément soit par administration d'un antigène protéique.

12. Animal transgénique non humain selon les revendications 9 ou 10, en outre transgénique pour une molécule du CMH de classe II humaine et, facultativement, en outre transgénique pour CD4 humain.

13. Inhibiteur de l'asparaginyl endopeptidase, qui a la structure B1-(XₐXₙ)-Asn-Q où B1 est tout groupe bloquant N-terminal approprié ; XₐXₙ sont les n résidus d'acides aminés immédiatement N-terminaux par rapport à un site de clivage Asn dans la chaîne invariante de molécules du CMH de classe II ; Asn est un résidu asparagine ; et Q est choisi dans les groupes constitués par les aldéhydes ; les halogénométhyl cétones ; la chlorométhylcétone ; la fluorométhylcétone ; le diazométhane (diazométhyl cétone) ; la vinyl sulfone-R (où R est tout groupe terminal alkyle ou aryle approprié et inclut alkyle en C₁ à C₁₀, phényle, benzyle, naphtyle et similaires) ; les (acyloxy)méthanes ; les N, O diacyl hydroxamates ; les nitriles ; les α-céto carbonyles ; les sels de céto-méthylsulfonium ; ou les époxydes ; et n est un entier de 1 à 100.

14. Inhibiteur selon la revendication 13, dans lequel le nombre de résidus d'acides aminés dans (XₐXₙ) est entre 1 et 25, de préférence entre 2 et 10.

15. Inhibiteur selon la revendication 14, qui est l'un quelconque parmi B1-Ser-Gln-Asn-Q ; B1-Leu-Glu-Asn-Q ; B1-Leu-Gln-Asn-Q ; B1-Pro-Glu-Asn-Q ; B1-Leu-Lys-Asn-Q ; B1-Gln-Asn-Q ; B1-Glu-Asn-Q ; B1-Asp-Glu-Asn-Q ; B1-Asn-Gly-Asn-Q ; B1-Phe-Pro-Asn-Q ; B1-Val-Pro-Asn-Q ; et B1-His-His-Asn-Q.

16. Inhibiteur de l'asparaginyl endopeptidase, qui a la structure (Xb-Xc)Asn(Xd-Xe) où (Xb-Xc) sont les r résidus d'acides aminés immédiatement N-terminaux par rapport à un site de clivage Asn dans la chaîne invariante de molécules du CMH de classe II et (Xd-Xe) sont les s résidus d'acides aminés immédiatement C terminaux par rapport à un site de clivage Asn dans ladite chaîne invariante; Asn est un résidu asparagine ; et r et s sont indépendamment entre 2 et 25, étant entendu que l'inhibiteur n'est pas le peptide CVFPNGTEVPNTRSRGHHN ou le peptide ATKYGNMTEDHVMHLLQNA.

17. Inhibiteur, qui a la structure (Xb-Xc)Asn(Xd-Xe) où (Xb-Xc) sont les r résidus d'acides aminés immédiatement N-terminaux par rapport à un site de clivage Asn dans la chaîne invariante de molécules du CMH de classe II et (Xd-Xe) sont les s résidus d'acides aminés immédiatement C terminaux par rapport à un site de clivage Asn dans ladite chaîne invariante ; Asn est un résidu asparagine ; et r et s sont indépendamment entre 2 et 5.

18. Inhibiteur selon l'une quelconque des revendications 13 à 15, ou inhibiteur qui a la structure (Xb-Xc)Asn(Xd-Xe) où (Xb-Xc) sont les r résidus d'acides aminés immédiatement N-terminaux par rapport à un site de clivage Asn dans la chaîne invariante de molécules du CMH de classe II et (Xd-Xe) sont les s résidus d'acides aminés immédiatement C terminaux par rapport à un site de clivage Asn dans ladite chaîne invariante ; Asn est un résidu asparagine ; et r et s sont indépendamment entre 2 et 25, pour une utilisation en médecine.

19. Procédé selon la revendication 1 ou utilisation selon les revendications 2 ou 3, dans lesquels l'inhibiteur est un inhibiteur selon l'une quelconque des revendications 13 à 18.
